# EUROPEAN PATENT SPECIFICATION

(11) **EP 1 592 377 B1**
(45) Date of publication and mention of the grant of the patent: **15.09.2010**
(21) Application number: 04711149.7
(22) Date of filing: 13.02.2004
(51) Int. Cl.: A61F 13/15, B65D 75/30

(54) **COMPACT ABSORBENT ARTICLE**
KOMPAKTER SAUGFÄHIGER ARTIKEL
ARTICLE ABSORBANT COMPACT

(30) Priority: 14.02.2003 US 366872
(43) Date of publication of application: 09.11.2005
(73) Proprietor: KIMBERLY-CLARK WORLDWIDE, INC., Neenah, WI 54956 (US)
(72) Inventor: KELLENBERGER, Stanley, R., Appleton, WI 54911 (US); RADWANSKI, Fred, R., Stone Mountain, GA 30087 (US); ROESSLER, Thomas, Harold, Appleton, WI 54915 (US); WEYENBERG, Roman, A., Jr., Neenah, WI 54956 (US); VELAZQUEZ, Herb, F., Neenah, WI 54956 (US); MCDONALD, Duane, L., Neenah, WI 54956 (US); NELSON, Denise, J., Hortonville, WI54944 (US)
(74) Representative: Davies, Christopher Robert
(86) International application number: PCT/US2004/004235
(87) International publication number: WO 2004/073571

(56) References cited:
- EP-A- 1 205 171
- WO-A-89/00037
- US-A1- 2002 079 246
- PATENT ABSTRACTS OF JAPAN vol. 1999, no. 09, 30 July 1999 (1999-07-30) & JP 11 113956 A (TOYO EIZAI CORP), 27 April 1999 (1999-04-27)

## Description

### BACKGROUND OF THE INVENTION

The present invention relates to a packaging of disposable absorbent articles. More particularly, the present invention relates to packaging of disposable absorbent articles such as diapers, training pants, adult incontinence products/garments, and so forth.

Absorbent articles such as, for example, diapers, training pants and incontinence garments desirably provide a comfortable fit about the wearer and contain body exudates. Such absorbent articles are commercially packaged in packages which include multiple articles therein.

Conventional diapers contained in a package are often folded such that each longitudinal side is folded inward, toward the crotch, and then the diaper is folded once, at the center so that it overlaps itself. Training pants and incontinence garments are often already configured to resemble underwear for the lower torso. These articles are also provided with few folds when positioned in a package of like products. Therefore, individual articles are relatively large and consume considerable space. Diapers and training pants are often carried in a diaper bag to accommodate a caregivers carrying a number of such articles. Incontinence garments are sized for adults, and present a greater problem regarding space and the desire to carry such garments in a discrete and confidential manner.

The disposable absorbent articles described above each contain an absorbent web which includes some amount of cellulosic absorbent material. Efforts to package one or a few such articles in a small, compact package have resulted in detrimental effects to the absorbent capabilities of the cellulosic and other absorbent material(s). This is because cellulosic material has little resiliency under certain normal conditions, and too much pressure or compression under those normal conditions removes the resiliency, resulting in stiffness and a reduction in performance. Such articles therefore have had an undesirable effect on ease of application, poorer fit, and are more likely to have an increase in undesirable leakage of exudates.

Continued space and modesty considerations, however, illustrate that a need exists for a compact package containing a single article which is provided in a much smaller, compact configuration compared to its expander use configuration. Such an article's performance desirably would not be substantially affected by its compact packaged configuration. When such an article was removed from its package and placed into its un-compacted use configuration, it would desirably provide the same performance via ease of application, fit, and absorbency and containment of exudates as an un-compacted article. Such a compact packaged article would desirably be sized to fit into a purse, or a standard shirt or pants pocket.

Prior art methods of folding garments are shown in JP 1111 3956, WO 89/00037 and EP1205171.

### SUMMARY OF THE INVENTION

There is provided, according to the present invention, a compact packaged disposable article as claimed in claim 1.

In the preferred embodiment of the invention, a compact packaged disposable absorbent article includes a disposable absorbent article. The disposable absorbent article has a folded configuration and an folded configuration. A ratio between the folded configuration and the unfolded configuration is less than 0.12. The compact disposable absorbent article also includes a package. The package has a gas permeability in a range of less than about 5.0 cc/100 in² (645.2 cm²)/24 hours to about 0.05 cc/100 in² (645.2 cm)/24 hours. The disposable absorbent article is folded to have more than three overlapping folds in its folded configuration. It is maintained and packaged in the package at a moisture content of less than 5 percent. The ratio between the folded configuration and the unfolded configuration of the article may be less than 0.09.

It is to be understood that both the foregoing general description and the following detailed description are exemplary and are intended to provide further explanation of the invention claimed. The accompanying drawings, that are incorporated in and constitute part of this specification, are included to illustrate and provide a further understanding of the articles and methods of the invention. Together with the description, the drawings serve to explain various aspects of the invention.

### BRIEF DESCRIPTION OF THE DRAWINGS

The present invention will be more fully understood and further advantages will become apparent when reference is made to the following detailed description of the invention and the accompanying drawings wherein like numerals represent like elements. The drawings are merely representative and are not intended to limit the scope of the appended claims.
Figure 1 representatively shows a plan view of one embodiment of a disposable absorbent article in an unfastened, laid flat with unretracted elastics and extended to ungathered length configuration with the surface of the article which contacts the wearer's clothing facing the viewer and with portions of the article partially cut away to show the underlying features;
Figure 2 representatively shows a perspective view of the absorbent article in a fastened configuration, generally similar to the configuration of the disposable absorbent article when it is fastened about a lower torso of a wearer;
Figure 3 representatively shows a plan view of the absorbent article in which each side is folded over the center;
Figure 4 representatively shows a perspective view of the absorbent article of Figure 3 folded into a bifolded configuration;
Figure 5 representatively shows a perspective view of the absorbent article of Figure 3 folded into a trifolded configuration;
Figure 6 representatively shows a perspective view of the absorbent article of Figure 4 folded into a quadrifolded configuration;
Figure 7 representatively shows a perspective view of the absorbent article of Figure 4 having each end folded over a center to provide generally an "S" configuration;
Figure 8 representatively shows a perspective view of the absorbent article of Figure 4 which has each end folded inward to provide generally a "Pretzel" configuration;
Figure 9 representatively shows a perspective view of the absorbent article of Figure 3 which has been rolled into a generally cylindrical configuration;
Figure 10 is a schematic representation of an apparatus for packaging disposable absorbent articles;
Figures 11A-11C representatively shows different embodiments of packages in which disposable absorbent articles may be packaged in accordance with the present invention;
Figure 12 representatively shows a perspective view of a child's training pant; and
Figure 13 representatively shows a perspective view of an adult incontinence pant or garment.

### DETAILED DESCRIPTION OF THE INVENTION

Reference will now be made in detail to one or more embodiments of the invention; examples of which are illustrated in the drawings. Each example and embodiment is provided by way of explanation of the invention, and not meant as a limitation of the invention. For example, features illustrated or described as part of one embodiment may be used with another embodiment to yield still a further embodiment. It is intended that the invention include these and other modifications and variations as coming within the scope and spirit of the invention.

It has been discovered that disposable absorbent articles, such as, by way of non-limiting example, a single disposable diaper, a single training pant, and a single adult incontinence pant or garment, may be compactly folded and packaged to provide a much smaller configuration or "footprint" when compared to its unfolded configuration or footprint, without compromising softness or absorbency. Cellulose and some super absorbent materials or particles (hereinafter collectively "SAP" or "SAPs"), such as, for example, partially neutralized and crosslinked polyacrylate, used in disposable absorbent articles hydrogen bond when brought in close proximity under certain normal conditions, such as when pressured or compressed when too moist, or when exposed to excessive relative humidity in the surrounding atmosphere while under a condition of being compressed. By controlling the moisture content of the absorbent core, temperature and pressure properly, it has been discovered that hydrogen bonding materials, such as, by way of non-limiting example, cellulose and SAPs, may be compactly folded and packaged for weeks, months, or years, yet still retain resiliency and performance when un-packaged for use without being stiff or having a significant reduction in the performance of the disposable absorbent article.

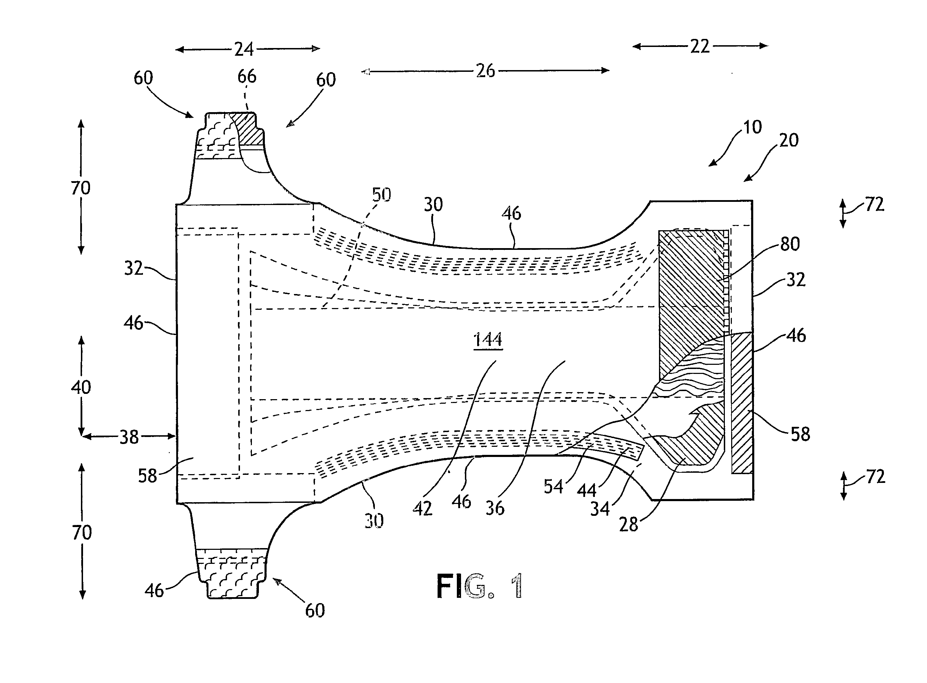

The unfolded configuration or footprint, as used herein refers to an absorbent disposable article, such as a diaper, positioned in its unfastened, laid flat with unretracted elastics and extended to ungathered lengths configuration, as best represented in Figure 1. Similarly, a pant-type disposable absorbent article 10, such as a training pant or an adult incontinence pant or garment is positioned by first cutting the article from each leg opening to the waist opening on each side (if the pant/garment is not provided with pre-existing openings in these areas), and positioning the article 10 in the unfolded and laid flat position described above. The folded compact configuration or footprint, as used herein, refers to a disposable absorbent article, such as a diaper, positioned in its folded compact position. Additional details of both configurations or footprints will be discussed in detail below. Methods of restraining and packaging such articles, by way of vacuum packaging, in combination with certain folding procedures and construction of the article, have been discovered. The results are a packaged compact disposable absorbent article that, when removed from its package, has substantially the same absorbency as those articles which have not been folded and packaged in a compact manner.

The compact absorbent articles of the present invention may include other types of absorbent articles, such as adult incontinent products, training pants, other personal care or health care garments, diaper pants and other types of garment-style disposable absorbent articles. Diapers are disclosed in U.S. Pat. No. 4,704,116 to Enloe, U.S. Pat. No. 4,050,462 to Woon et al., U.S. Pat. No. 5,383,872 to Roessler et al., U.S. Pat. No. 6,028,240 to Wessel et al., U.S. Pat. No. 6,316,687 to Davis et al., and U.S. Pat. No. 6,443,938 to Vogt. Training pants are disclosed in U.S. Pat. No. 4,641,381 to Heran et al., U.S. Pat. No. 4,646,362 to Heran et al., U.S. Pat. No. 5,766,389 to Brandon et al., and U.S. Pat. No. 6,297,424 to Olson et al. Adult incontinence products are disclosed in U.S. Pat. No. 4,630,320 to Van Gompel, U.S. Pat. No. 4,886,512 to Damico et al., U.S. Pat. No. 5,651,778 to Melius et al., U.S. Pat. No. 6,260,211 to Rajala et al., and U.S. Pat. No. 6,387,085 to Van Gompel et al.

Specifically, the present invention is directed toward disposable folded compact absorbent articles which are used at least, but not by way of limitation, to absorb urine, and which are designed to cover at least a portion of a lower torso when worn by a wearer. That is, the article is configured to extend about the circumference of the lower torso and between the legs of a wearer when worn. It will be appreciated, however, that descriptions, figures, examples, test(s), and so forth of a particular packaged compact disposable article, such as a diaper, are only by way of example, and are intended as non-limiting in the scope and spirit of the invention.

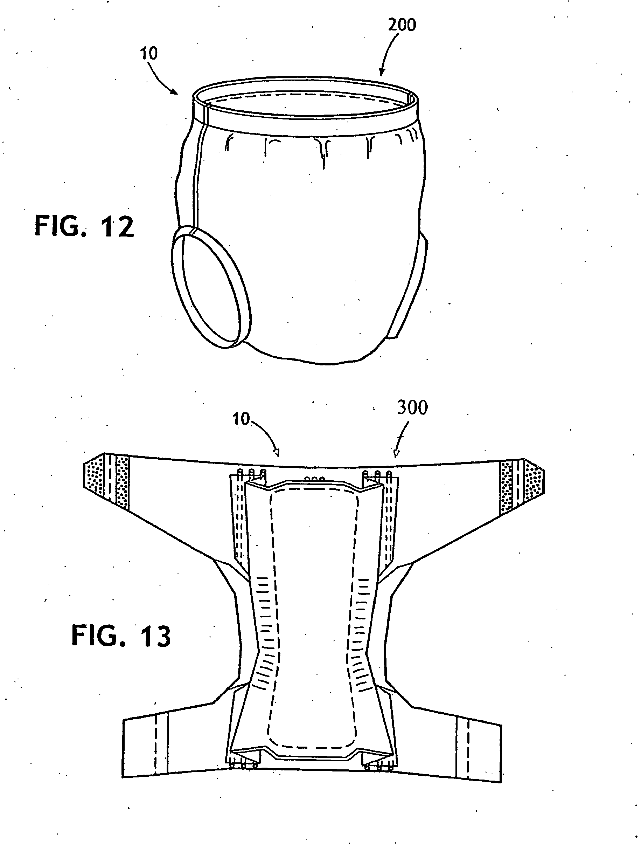

An embodiment of a disposable absorbent article 10, is illustrated in Figure 1, in this instance, a diaper 20. Figure 12 shows another example of a disposable absorbent article 10, in this instance, a child's training pant 200. Figure 13 illustrates yet another example of a disposable absorbent article 10, in this instance, a refastenable adult incontinence pant or garment 300.

Turning now, however, to Figure 1, the diaper 20 is shown in an unfastened, laid flat with unretracted elastics and extended to ungathered length configuration with the surface of the diaper 20 adapted to contact the wearer's clothing facing the viewer and with portions of the diaper 20 partially cut away to show the underlying features. The illustrated diaper 20 defines a front waist region 22, a back waist region 24, a crotch region 26 that extends between and connects the front and back waist regions 22 and 24, a longitudinal direction or length dimension 38 and a lateral direction or width dimension 40. As used herein, the term "longitudinal direction" means the direction that is parallel to the long dimension (usually machine direction) of the diaper 20 and generally corresponds to the "y" direction of the diaper 20. As used herein the term "lateral direction" means the direction that coincides with the width direction which generally is perpendicular to the longitudinal of machine direction of the diaper 20 and generally corresponds to the "x" direction of the diaper 20.

Measurements of the area immediately within the outer perimeter or outer periphery 46 of the diaper 20 along the longitudinal direction 38 and the lateral direction 40 provide the area (Table 1) for the unfolded configuration or footprint of the diaper 20. The method of measurement is discussed in detail below in Example 1 A.

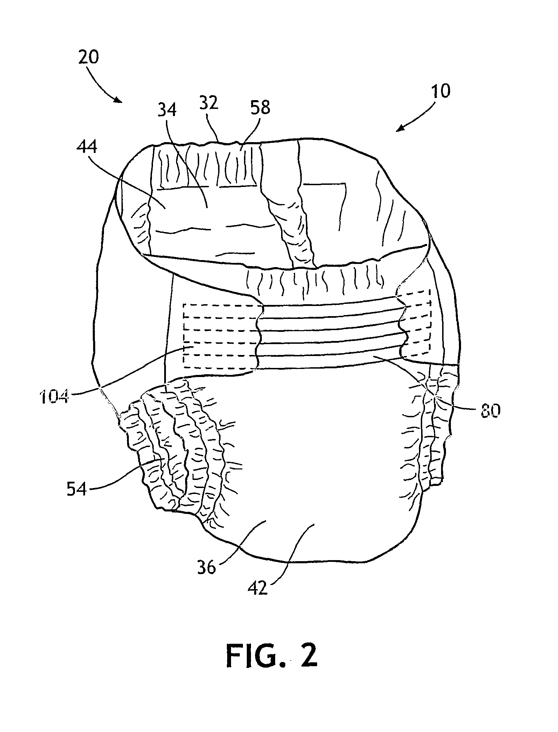

The front waist region 22 includes the portion of the diaper 20 which, when worn, is positioned on the front of the wearer. The front waist region 22 further defines front ear regions 72 generally in the laterally outward portions of the front waist region 22. The back waist region 24 comprises the portion of the diaper 20 which, when worn, is positioned on the back of the wearer. The back waist region 24 further includes back ear portions 70. When the diaper 20 is worn, back ear portions 70 are overlapped over front ear regions 72, as shown in Figure 2. The crotch region 26 of the diaper 20 includes the portion of the diaper 20 which, when worn, is positioned between the legs of the wearer and covers the lower torso of the wearer.

The diaper 20 defines a pair of laterally opposed side edges 30, a pair of longitudinally opposed waist edges 32, an interior surface 34 (facing away from the viewer) which is configured to contact the wearer, and an exterior surface 36, opposite the interior surface 34, which is configured to contact the wearer's clothing in use. The illustrated diaper 20 also includes an outer cover 42 and a bodyside liner 44 which is connected to the outer cover 42 in a superposed relationship and an absorbent core 28. The absorbent core 28 is located between the outer cover 42 and the bodyside liner 44. The laterally opposed side edges 30 of the diaper 20 are generally defined by the side edges 30 of the outer cover 42 which further define leg openings that are formed when the article is worn and may be curvilinear. The waist edges 32 of the diaper 20 are generally defined by the waist edges 32 of the outer cover 42 and define a waist opening which is configured to encircle the waist of the wearer when worn. The absorbent core 28 is configured to contain and/or absorb any body exudates discharged from the wearer. The diaper 20 may further include leg elastics 54, containment flaps (not shown) and waist elastics 58 as are known to those skilled in the art. It should be recognized that individual components of the diaper 20 may be optional depending upon the intended use of the diaper 20.

The diaper 20 may be of various suitable shapes. For example, in the unfastened configuration as illustrated in Fig. 1, the diaper 20 may have an overall rectangular shape, T-shape or a generally I-shape. In the embodiment shown in Fig. 1, the diaper 20 has an approximately hourglass shape in an unfastened configuration.

Examples of diaper configurations suitable for use in connection with the instant application and other diaper components suitable for use en diapers are described in U.S. Patent 4,798,603 issued January 17, 1989, to Meyer et al.; U.S. 5,176,668 issued January 5, 1993, to Bernardin; U.S. 5,192,606 issued March 9, 1993, to Proxmire et al., and U.S. 5,509,915 issued April 23, 1996, to Hanson et al.

The bodyside liner 44, as representatively illustrated in Fig. 1, suitably presents a bodyfacing surface which is compliant, soft feeling, and nonirritating to the wearer's skin. Further, the bodyside liner 44 may be less hydrophilic than the absorbent core 28, to present a relatively dry surface to the wearer and to isolate the wearer's skin from liquids held in the absorbent core 28.

The absorbent core 28 of the diaper 20, as representatively illustrated in Fig. 1, may suitably include a matrix of hydrophilic fibers, such as a web of cellulosic fluff, mixed with particles of a high-absorbency material commonly known as superabsorbent material (SAPs); in a particular aspect, the absorbent core 28 includes a matrix of cellulosic fluff such as wood pulp fluff and superabsorbent hydrogel-forming particles, namely, SAPs. The wood pulp fluff may be exchanged with synthetic, polymeric, meltblown fibers or with a combination of meltblown fibers and natural fibers. The SAPs may be substantially homogeneously mixed with the hydrophilic fibers or may be nonuniformly mixed. The fluff and SAPs may also be selectively placed into desired zones of the absorbent core 28 to better contain and absorb body exudates. The concentration of the SAPs may also vary through the thickness of the absorbent core 28. Alternatively, the absorbent core 28 may include a laminate of fibrous webs and SAPs or other suitable means of maintaining SAPs in a localized area.

The absorbent core 28 may have any of a number of shapes. For example, the absorbent core 28 may be rectangular, I-shaped (including an hour glass shape, or a shape which is expanded outward at one or both ends), T-shaped, and so forth. It is generally preferred that the absorbent core 28 be narrow in the crotch region 26 of the diaper 20. The size and the absorbent capacity of the absorbent core 28 should be compatible with the size of the intended wearer and the liquid loading imparted by the intended use of the absorbent article.

The high absorbency material or particles, i.e., SAPs, may be in any of a wide variety of geometric forms. As a general rule, it is preferred that the high absorbency material be in the form of discrete particles, SAPs, which may take any of a number of shapes. The SAPs are generally present in the absorbent core 28 in an amount of from about 5 to about 90 weight percent based on total weight of the absorbent core 28. Desirably, the SAPs are present in the absorbent core 28 in an amount of from about 20 to about 90 weight percent based on the total weight of the absorbent core 28. More desirably, the SAPs are present in the absorbent core 28 in an amount of from about 30 to about 90 weight percent based on the total weight of the absorbent core 28. Yet even more desirably, the SAPs are present in the absorbent core 28 in an amount of from about 40 to about 90 weight percent based on the total weight of the absorbent core 28. SAPs are well known to those skilled in the art and are widely commercially available.

A fastening system helps maintain the initial fastened position of the diaper 20 during use so as to maintain proper tension and ultimately proper fit. As illustrated in Figs. 1 and 2, the diaper 20 includes the back ear portion 70, which has fasteners 60. The fasteners 60 may include an attachment portion 66, such as an adhesive, desirably a pressure sensitive adhesive. Alternatively, the attachment portion 66 may include a hook material, and so forth. The front waist region 22 includes a landing zone 80. If the attachment portion 66 of the fastener 60 is an adhesive, the landing zone desirably includes a material configured to receive and hold an adhesive fastener thereto. Alternatively, is the attachment portion 66 of the fastener 60 is a hook material, the landing zone desirably includes a loop material for releasably coupling the hook material thereto. In use, the caregiver overlaps each back ear portion 70 and fastener 60 over the front waist region 22 and engages the fasteners 60 to the landing zone 80, as illustrated in Figure 2, creating an overlap region 104 which holds the diaper 20 in position about a torso.

The diaper 20 has been discussed in detail herein. However, it will be understood by those skilled in the art that the foregoing description also applies to a training pant 200 (Figure 12) and to an adult incontinence pant or garment, whether formed as a pant (not shown) or provided as a refastenable garment 300 (Figure 13).

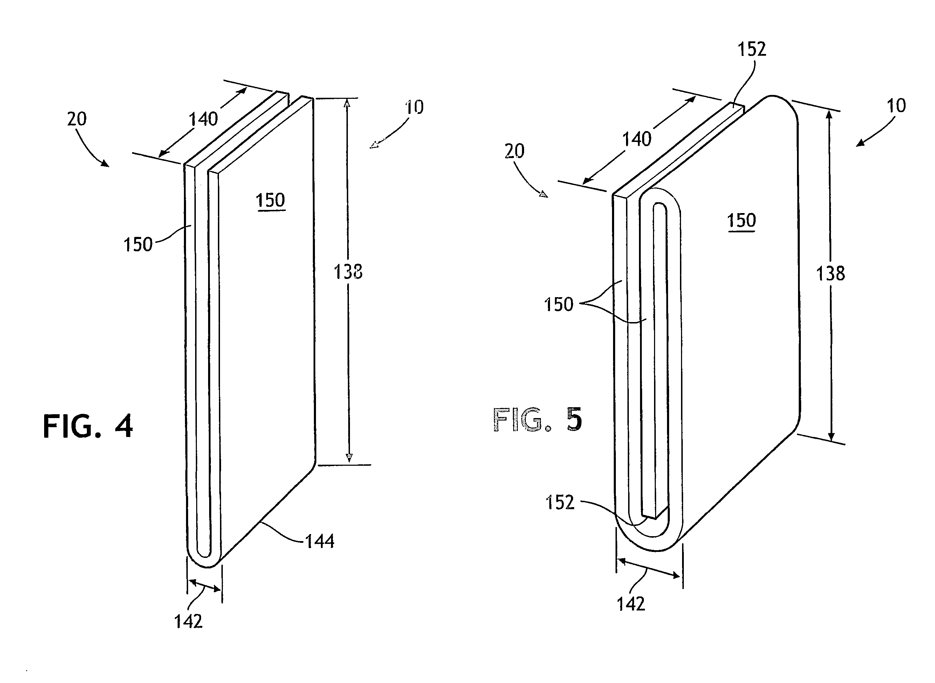

Turning now to folding configurations or "footprints", a disposable absorbent article 10, that is, for purposes of illustration only and not by way of limitation, the diaper 20 illustrated in Figures 1 and 2, is folded to provide a reduced or more compact configuration or footprint. The various compact footprints, created by different folding techniques, are compared to the unfolded footprint, that is, the measurement of the area as defined immediately within the outer perimeter or outer periphery 46 of the diaper 20, as described in detail in Example 1A, and the results are provided in Table 1. Each folded compact configuration of each article 10, namely, the diaper 20 described below and shown in Figures 4-9 is measured along a longitudinal direction or length dimension 138 (generally along a "y" direction) and a lateral direction or width dimension 140 (generally along an "x" direction).

In addition, a depth dimension 142 (generally along a "z" direction, generally positioned perpendicularly relative to a "y" direction), is measured in both the unfolded footprint of the diaper 20, and the folded compact footprint. The description of the measurement of the depth dimension will be described in the Examples, below. Therefore, area and volume calculations of each folded article compared to the unfolded article are provided in Table 1.

Folds in disposable absorbent articles containing highly absorbent materials have been problematic. Fords have created problems in absorption in the folded area, and for this reason have traditionally been kept to a minimum. Folds cause compacting of the cellulosic material in the inside of the folded "crease," and tend to cause movement (stretching) of the absorbent materials on the outside of the fold toward either side of the fold. This results in a reduction in the absorbent capabilities of both the cellulosic material and the highly absorbent materials in and around the folded area. Not to be constrained to this explanation, one mechanism of this reduction of absorbent capability is believed to be due to discontinuities in the absorbent composite caused by this stretching and compacting.

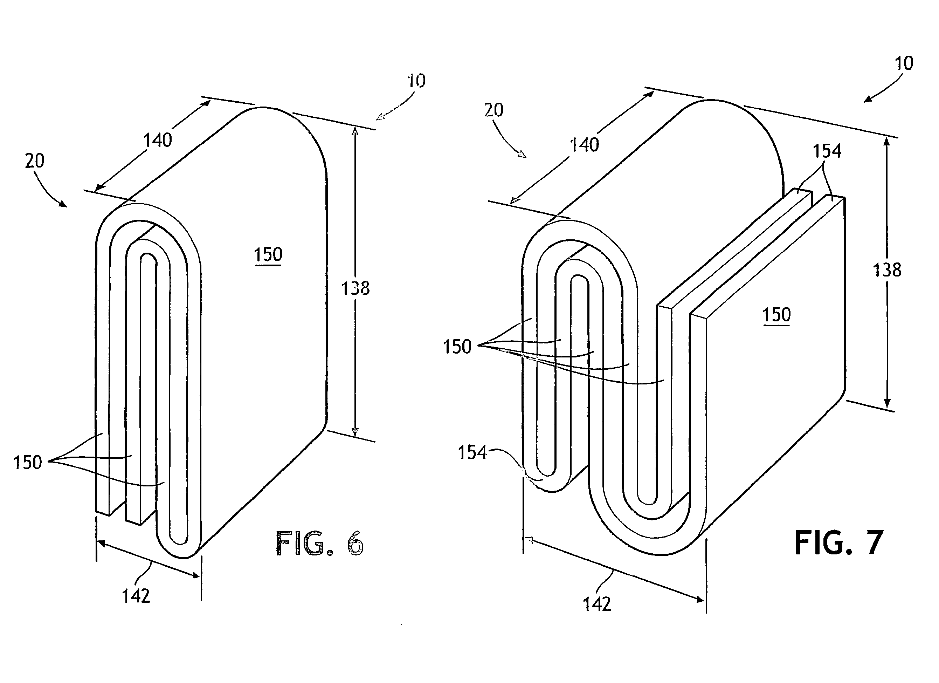

Folds in the present invention have been created which work with existing folded patterns, in order to minimize the sharp angle of additional folds. It is believed that folds, such as those illustrated in Figures 6-8, which are created after the initial bifolding of the diaper 20, as shown in Figure 4, have a larger radius of curvature, which reduce or eliminate the disadvantages described previously for folds having a sharp angle or small radius of curvature. "Folds" or "folded configuration" as used herein refers to at least one planar surface followed by at least one radius of curvature.

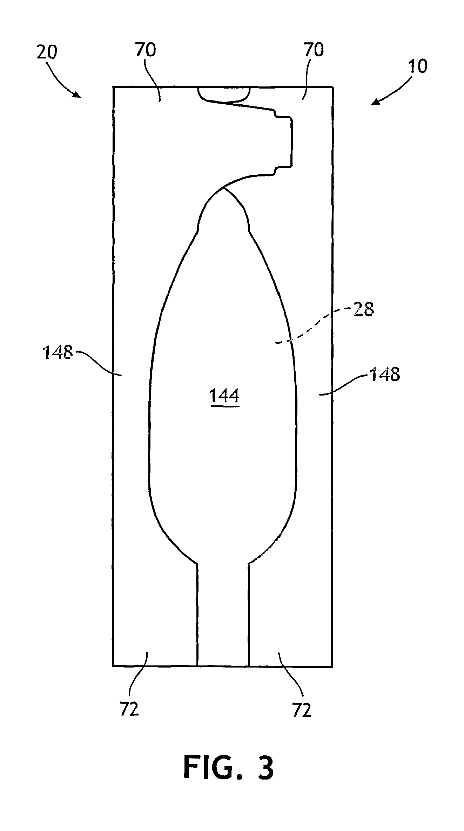

In initially folding a disposable absorbent article 10 such as, by way of non-limiting example, a diaper 20, each side 148 is folded inward, including the back ear portions. 70 and the front ear regions 72, over the area containing the absorbent core 28, as shown in Figure 3. The diaper 20 is then folded again at approximately the center 144 to provide a bifolded diaper 20 having two overlapping panels 150 of approximately equal length, as schematically illustrated in Figure 4.

In an alternative, as schematically shown in Figure 5, the diaper 20, folded initially as shown in Figure 3 is folded such that each end 152 is folded over and overlaps about a third of the other to provide three approximately equal and overlapping panels 150. In this manner, a trifolded diaper 20 is provided.

In another alternative, as schematically illustrated in Figure 6, the bifolded diaper 20 shown in Figure 4 is then overlapped again to provide a quadrifolded diaper 20 having four approximately equal and overlapping panels 150.

In yet another alternative, as schematically illustrated in Figure 7, the bifolded diaper 20 shown in Figure 4 is then folded such that approximately one third of each bifolded end 154 of the folded diaper is folded over each side of a center portion to provide an "S" folded diaper having six approximately equal and overlapping panels 150.

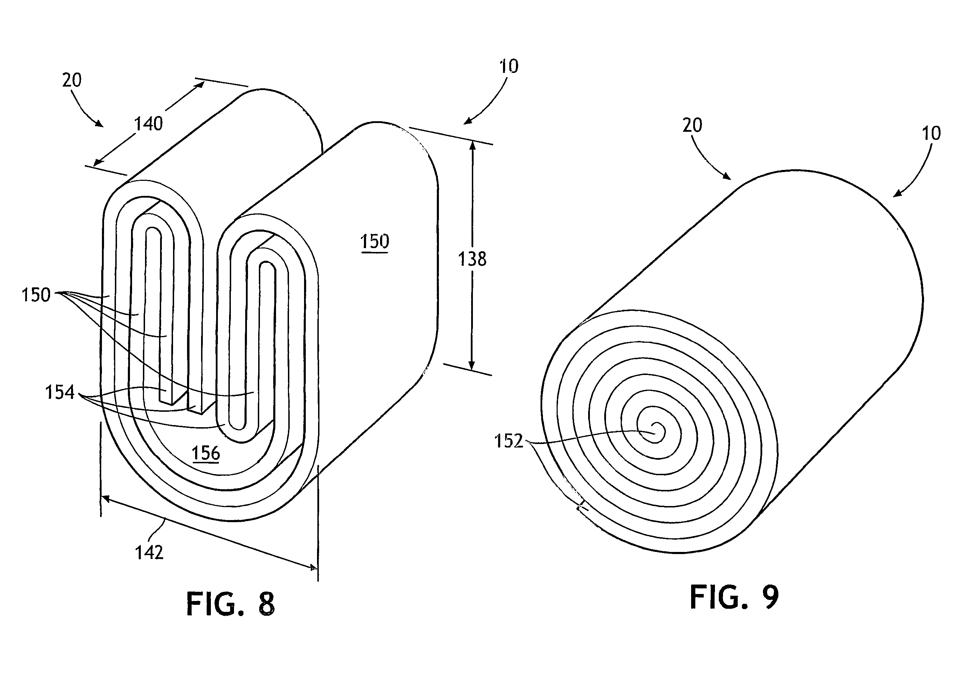

In still yet another alternative, as schematically illustrated in Figure 8, each end 154 of the bifolded diaper 20 shown in Figure 4 is overlapped inwardly, toward a center portion 156 to provide a "Pretzel" fold having eight overlapping panels 150.

In an arrangement, not according to the present invention, illustrated in Figure 9, the diaper 20 of Figure 3 is rolled from one end to the other to form a generally cylindrical shape. This configuration, providing a continuous rotation of the diaper 20 around a center, differs from folds; it has no planar surface in the folded configuration, other than each end, since it forms, generally, a cylindrical shape. It will be understood, however, that the present article 10 may be folded in any manner consistent with the present invention, and the foregoing folding techniques.

It will also be understood that the article 10 is folded such that pressure to the absorbent material is impacted as little as possible. That is, folds are placed longitudinally along either side of the crotch. Folds in the crotch area generally have a greater radius of curvature and are positioned in an area of the article 10, that is, the diaper 20 in this instance, which would naturally bend around the body of a wearer and/or to provide ease of unfolding by a caregiver. Scoring or embossing the portions of the article 10 along longitudinal fold lines and near the waist would prevent cracking, and provide some barrier properties, which would be desirable especially at the waist area. Rolling the article 10, as shown generally by the diaper 20 in Figure 9 with the thinnest portion, that is, the outer cover 42 of the diaper 20 positioned toward the inside of the roll, increases the radius of curvature of the thicker portion thus reducing the likelihood of cracking.

In folding the article 10, folding the fasteners 60 must be taken into consideration. Fasteners 60 desirably are not folded in the width 40 (cross-machine) direction, because such folding impacts the ability of the attachment portions 66 to fasten to the landing zone 80. Damage to the attachment portions 66 may occur if the fastener 60 is bent such that a portion of the fastener 60 lies in one plane while another portion lies in a curve and/or another plane. Specifically, the attachment portions 66 may be damaged such that they are not capable of maintaining a sufficient contact with the landing zone 80, thereby causing the article 10 to unfasten from a torso. Therefore, the fasteners 60 are desirably maintained in a consistent plane or planar surface when folded with the article 10 in any of the foregoing folded configurations so that the fastener 60 and attachment portion 66 thereof is lying substantially within the same plane.

The article 10 may be folded by hand, or it is desirably machine folded (not shown). The article 10 is then packaged.

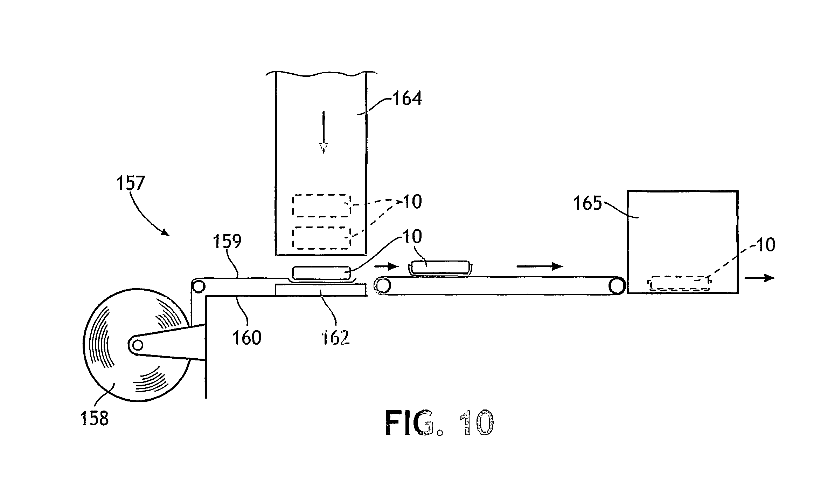

Schematically shown in Figure 10 is one method and apparatus 157 of packaging an article. In this example, a first reel 158 has sheet material 159 which is rotatably supported on a bed 160 having a vacuum cavity former 162 provided where the article 10 is disposed. Articles 10, such as, for example, the diaper 20 folded as illustrated in Figure 7, are provided at a loading station 164 and they are disposed on the sheet material 159 over the cavity former 162. The articles 10 are carried to a packaging station 165 which has a vacuum chamber (not shown). The packaging station 165 also contains film (not shown) and a sealing apparatus (not shown) for sealing the articles 10 in the cavities while they are in the vacuum chamber. The articles 10 in the forming cavities 162 are carried into the packaging station 165 and the chamber is closed. A vacuum is applied to the articles 10 and the cavities 162, and film is then positioned and sealed over each cavity via sealing devices, such as thermal sealing, ultrasonic bonding, or any other sealing methods known by those skilled in the art. The vacuum depressurizes the chamber in the packaging station to a pressure which is less than the atmospheric pressure outside of the chamber. After the package is sealed, the vacuum is removed from the chamber, and the chamber and the articles sealed in packages therein are permitted to return to regular atmospheric pressure, which results in compression of the articles in the packages, providing a soft package (form-fill-seal) which is formed about the article 10. The packaged article 190 may thereafter be removed (not shown). It will be appreciated that many techniques and apparatus for vacuum packaging are known to those skilled in the art, and are commercially available. Any type of packaging device may be utilized which permits the article 10 to perform in the manner described and illustrated herein. Another type of vacuum packaging is disclosed, for example, but not by way of limitation, in U.S Pat. No. 4,833,862 to Bortolani, et al. Many techniques and apparatus for vacuum packaging are known in the art and commercially available.

The amount of vacuum is an amount necessary to result in compression of the article so that it may be contained and reduced in size within and along with the package, but not so much as to disrupt elements of the article, such as an amount which would displace the SAPs or overly compress the cellulosic material. The amount of vacuum is generally in a range of about 32 (81.3 cm) to about 5 inches (12.7 cm) of Mercury. Desirably the range is about 30 (76.2 cm) to about 8 inches (20.3 cm) of Mercury. Even more desirably, the range is about 28 (71.1 cm) to about 10 inches (25.4 cm) of Mercury. Yet even more desirably, the range is about 27 (68.6 cm) to about 15 inches (38.1 cm) of Mercury. Still yet even more desirably, the range is about 26 (66.0 cm) to about 18 inches (45.7 cm) of Mercury. However, yet even more desirably, the range is about 25 (63.5 cm) to about 20 inches (50.8 cm) of Mercury.

It has been discovered, however, with regard to the articles 10, namely, the diaper 20 illustrated in Figures 6-9, that when folded, it tends to spring back out of the desired folded configuration prior to being sealed in a package. That is, the article 10 tends to move or "spring back" out of its desired folded configuration, and return back to its previous configuration. To eliminate this problem, the cavity for the package was sized slightly smaller, i.e., about 0.1 (0.25 cm) to about 0.5 inches (1.27 cm), than the length dimension 138 and/or the width dimension 140 of the folded diaper 20. This slight reduction in the cavity dimension acts to minimize the spring back so that the article 10 is retained in its desired configuration or footprint until sealed. Alternatively and/or in addition to, a weight may be applied temporarily to the folded article 10, to induce some "memory" of the desired configuration until sealed in a package. In another alternative, an adhesive may be applied to a portion(s) of the article 10 prior to folding, to assist in holding it in the desired folded configuration. A pressure sensitive adhesive may be used, or a cohesive adhesive, such as, by way of non-limiting example, latex, may be utilized. The adhesive may be applied in spots, strips, patterns, decorations, and so forth. Further, the adhesive may be clear and colorless, and applied over a decoration(s) on the article to "hide" the adhesive. In another alternative, the adhesive may be colored, and may be applied as part or all or a decoration(s) on the article, In yet another alternative, the article 10 may be folded into the desired configuration and banded by one or more bands which extend about the perimeter of the article 10.

In the embodiment of Figure 10, the package is at least partially a preformed package. The compression or pressure on the article may be exerted at least partially by the package. Alternatively, the package may be formed around the article 10.

The article, vacuum packaged, is sealed in a package desirably constructed of a material with a substantially low gas permeability, including, but not limited to, a polymeric film, such as, by way of non-limiting example, a polyethylene terephthalate (PET), a polyvinyldichloride (PVDC), having an oxygen transmission of about less than 3.0 cc/100 in² (645.2 cm²)/24 hours @ 73 degrees F. (22.78 °C) and 0 percent relative humidity (RH). Multilayer films, each providing a different function, may be utilized. For example, one layer may have a heat sealable property, such as polypropylene or polyethylene, another layer may provide strength, such as polyester and/or nylon, and another layer may provide substantially reduced gas permeability, such as PET or PVDC. Also, films with reduced permeability to vapors (that is, films that have substantially low gas permeability or are substantially gas impermeable for a variety of applications) can themselves be composites, such as where one layer is a flexible polymer, such as polyurethane, polyethylene, ether polyurethane, or polypropylene, while another layer is coated on or coextruded and serves as a barrier layer. Barrier layers can generally be viewed as substantially organic based or substantially inorganic based. For example, U.S. Pat. No. 3,442,686, describes a film composite in which silicon oxide coatings are deposited on polymers to serve as a barrier layer. This produces barrier coatings on even quite thin polymer films of oxygen transmission rate properties of about 0.2 cc/100 in² (645.2 cm²) day and similar water vapor transmission rate properties. However, it will be appreciated that any material having substantially low gas permeability or substantially gas impermeable material(s) known in the art may be used.

With a substantially low gas permeable material and/or package, the gas permeability rate is less than about 5.0 cc/100 in² (645.2 cm²)/24 hours to about 0.05 cc/100 in² (645.2cm²)/24 hours. Desirably, the gas permeabitity rate is less than 4.0 cc/100 in² (645.2cm²)/24 hours to about 0.07 cc/100 in² (645.2cm²)/24 hours. More desirably, the gas permeability rate is less than about 3.0 cc/100 in² (645.2 cm²)/24 hours to about 0.10 cc/100 in² (645.2 cm²)/24 hours. Even more desirably, the gas permeability rate is less than about 2.0 cc/100 in² (645.2 cm²)/24 hours to about 0.10 cc/100 in² (645.2 cm²)/24 hours.

A number of different packages may be utilized which are suitable for use with the present invention. Types of packages which are particularly suitable include, but are not limited to form-fill-seal packages, blister packages, injection molded packages (with and without living hinges), heat sealed pouches, thermally formed trays with lids, and any suitable package known to those skilled in the art.

Packaging the article using a substantially gas impermeable material permits the moisture content to be maintained. Most importantly, the moisture content can be maintained at a relatively low level when hydrogen bonding type materials, e.g., cellulose and SAPs are present, as they are in the articles referenced herein. That is, inhibiting an increase in moisture while maintaining the article in its folded, compact, compressed and packaged state eliminates detrimental hydrogen bonding from taking place that would have occurred if the moisture level in the absorbent core was too high at the time the article was packaged or during packaging, or if the article was packaged in a package having one or more openings in it.

Before the articles are packaged, and when the articles are packaged, it is desirable to maintain a lower moisture level. High moisture levels result in hydrogen bonding in the absorbent core of the article. Therefore, the articles are desirably maintained and packaged with an absorbent core at a moisture level of less than 5%. Even more desirably, the articles are maintained and packaged with an absorbent core at a moisture level of less than 3%.

In addition, it is advantageous to control heat during folding and packaging the article. The temperature of at least the absorbent core is desirably kept below about 110 degrees C. Even more desirably, the temperature of at least the absorbent core is kept at below about 80 degrees C. Yet even more desirably, the temperature of at least the absorbent core is kept below about 50 degrees C. Still even more desirably, the temperature of at least the absorbent core should be kept below about 24 degrees C.

In addition, it is noted that vacuum packaging assists in preserving desired characteristics of the absorbent web materials, particularly crosslinked cellulosic curly fibers and even more so non-crosslinked cellulosic fibers by maintaining their stiffness and resiliency. Vacuum packaging is advantageous by providing pressure which is applied relatively uniformly to the article. Vacuum packaging, even though the article is folded, creates fewer hard, highly bonded areas of the absorbent core (for example, in the target area of the absorbent core 28) will not be at a higher density than the low basis weight areas (for example, the back of the absorbent core 28 the article 10).

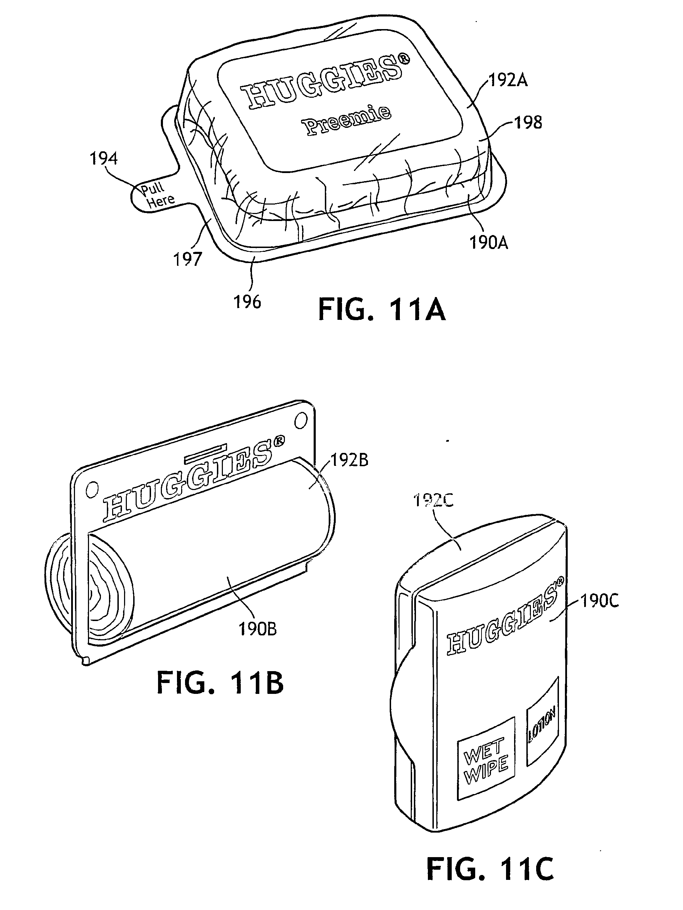

Figures 11A -11C illustrate several different packaged compact absorbent articles 190. Package 192A illustrates a form-fill-seal package holding a compact absorbent article 190A (such as, for example, the article shown in Figure 7), as also generally shown in Figure 10. Package 192B, as shown by Figure 11B, shows a blister package having a living hinge and holding a compact absorbent article 190B (such as the article shown in Figure 9). Figure 11C illustrates an injection molded package having a living hinge (not shown) which holds a compact absorbent article 190C. Each package 192A, 192B, and 192C is intended, without limitation, to illustrate some of the possible embodiments of packages which are suitable for use with the present invention, however, it will be understood that any package known in the art may be used. The packages 190A-190C desirably include curvilinear corners, and each desirably provides size information (Figure 11A) for the compact absorbent article contained therein, as well as one or more attractive decorations, such as one or more letters, numbers, symbols, designs and/or patterns, an area to write a user's name and other information, and so forth. In addition, a wet sheet packet, a lotion pocket, a changing sheet, a disposal bag, one or more coupons, and so forth may be included on an inside of the package, as illustrated in 190C, or on an outside of the package (not shown). Desirably, each package 190A-190C will open without any small pieces of the package tearing away separately, which is undesirable in the presence of small children and infants. The package may be provided such that a plurality of single packaged, articles are releasably coupled together by, for example, but not pay way of limitation, perforations, bands, adhesives, and so forth.

Further desirably, as illustrated in Figure 11A, the package 192A will have a pull tab 194. The pull tab 194 desirably extends beyond a perimeter 196 of the package 192A, so that it is easy to grasp between a thumb and a finger by a caregiver, and permits easy opening of the package while maintaining the package 192 in one piece after being opened, and after the article has been removed.

The pull tab 194 shown in Figure 11A may be formed as a portion of the top material 197 of the package 192A, and is desirably, but not by way of limitation, not coterminus with the bottom material 198 of the package 192A to which the top material 192 is sealed. The pull tab 194 may have pull indicia such as words, symbols, and so forth (for example, but not by way of limitation, "Pull Here" illustrated in Figure 11A). The pull tab 194 may be formed of a thicker material, or may be embossed or otherwise textured, and so forth, to provide easy grasping and pulling by a caregiver. The pull tab 194 may be formed in one or more corners of the package 192A, or one or more pull tabs 194 may be provided on any portion(s) of the package 192A (not shown). Desirably, the edges of the pull tab 194 are curvilinear.

Ideally, the compact absorbent article 190, when packaged, will have a configuration that permits it to be placed in a purse, a jacket pocket, a pants pocket, or a shirt pocket. Each type of pocket has somewhat different dimensions, but generally, it is desirable to have an article 190, for example, a single packaged diaper, which has a width dimension of less than 6 inches (15.2 cm), a length dimension of less than 11 inches (27.9 cm), and a depth dimension of less than 1.75 inches (4.44 cm). More desirably, the article 190 will have a width dimension 138 of less than 5 inches (12.7 cm), a length dimension 140 of less than 5.5 inches (13.97 cm), and a depth dimension of less than 1.5 inches (3.81 cm). Even more desirably, the article 190 will have a width dimension of less than 4.6 inches (11.68 cm), a length dimension of less than 4.0 inches (18.2 cm), and a depth dimension of less than 1.75 inches (4.44 cm). Yet even more desirably, the article 190 will have a width dimension of less than 4.6 inches (14.7 cm), a length dimension of less than 3.8 inches (9.65 cm), and a depth dimension of less than 1.3 inches (2.30 cm). Such ranges described herein provide a footprint, that is, an area, or projected area, as well as a volume for the packaged compact absorbent article 190 so that it easily fits into a shirt pocket, a pants pocket, a jacket pocket, a small purse, and so forth, to provide ease in carrying and concealing the article 190.

The area or footprint of the folded compact absorbent article 190 is compared to the unfolded and laid flat with unretracted elastics and extended to ungathered length configuration of the article 10 (as exemplified by the disposable diaper 20), is provided in Table 1. Table 1 provides the area for the unfolded and laid flat article 10 positioned as described above, as well as the area for each different type of folded compact absorbent article 190. According to the present invention, the ratio between the folded configuration and the unfolded configuration is less than 0.12. Desirably, the ratio between the folded configuration and the unfolded configuration is less than 0.11. However, more desirably, the ratio between the folded configuration and the unfolded configuration is less than 0.10. Even more desirably, the ratio between the folded configuration and the unfolded configuration is less than 0.09. Still yet even more desirably, the ratio between the folded configuration and the unfolded configuration is less than 0.07. Yet even more desirably, the ratio between the folded configuration and the unfolded configuration is less than 0.05.

The volume of the compact absorbent article is desirably less than 30 in³ (491.6 cm³) More desirably, the volume of the compact absorbent article is less than 15 in³ (245.8 cm³). Even more desirably, the volume of the compact absorbent article is less than 12 in³ (196.6 cm³). Yet even more desirably, the volume of the compact absorbent article is less than 10 in³ (163.9 cm³). Still yet even more desirably, the volume of the compact absorbent article is less than 7 in³ (114.7 cm³). However, yet even more desirably, the volume of the compact absorbent article is less than 5 in³ (81.9cm³).

When an article 10 is packaged, that is vacuum packaged, it is expected that its size will be reduced in width and length i.e., in two dimensions. As measured in two dimensions, width and length (area - inches²) and compared against a substantially similarly folded un-packaged article (Example 1 and Example 4D), the article 10 is desirably reduced in area by more than about 10 percent. Even more desirably, the article 10 is reduced in area by more than about 12 percent. Yet even more desirably, the article 10 is reduced in area by more than about 15 percent. Still yet even more desirably, the article 10 is reduced in area by more than about 17 percent. However, even more desirably, the articles 10 is reduced in area by more than about 20 percent. Yet even more desirably, the article 10 is reduced in area by more than about 22 percent.

When the article 10, as described immediately above is packaged and measured in three dimensions I.e., width, length and depth (volume - inches³) and compared against a substantially similarly folded un-packaged article 10 (Example 1 and Example 4D); the article 10 is desirably reduced in volume by more than about 10 percent. Even more desirably, the article 10 is reduced in volume by more than about 15 percent. Yet even more desirably, the article 10 is reduced in volume by more than about 20 percent. Still yet even more desirably, the article 10 is reduced in volume by more than about 30 percent. However, even more desirably, the article 10 is reduced in area by more than about 40 percent. Yet even more desirably, the article 10 is reduced in volume by more than about 45 percent. As described below in the TEST, this two and three dimensional reduction (area and volume) resulted in no significant changes in performance of the article. Throughout the following Examples and Test, the non-metric units pounds ('lbs') and inches ('"') are used. It should thus be noted that one inch = 2.54 cm, one square inch = 6.45 cm², one cubic inch = 16.387 cm³ and one pound = 0.4536 kg.

### EXAMPLES

### Example 1

A diaper (HUGGIES® ULTRATRIM® Step 3 diaper, available from Kimberly-Clark Corporation, Neenah, WI) was produced which is similar to the article illustrated in Figure 1, and described in detail herein. The diaper was configured to fit an infant weighing about 16 to about 28 pounds. The diaper was folded by hand as illustrated in Figure 7 and described previously in detail herein, in the "S" fold configuration, and positioned in a cavity formed from a material, namely, 611K-3 mil nylon with LLDPE coextrusion, available from Curwood, New London, WI, having at least one layer of a substantially low gas permeability or one layer of a substantially gas impermeable material, which was formed and positioned in a horizontal form/fill/seal packaging machine, in this instance, a RapidPak RP-55, manufactured by Alkar-Rapidpak, Inc., Lodi, WI. The top material, that is, the sealing cover, also having at least one substantially gas impermeable layer, was a 48 gauge polyester film with 2 mils HDPE peel seal (coextrusion), available from Curwood, New London, WI. The top material was provided as a "peel back" material for a caregiver's convenience, and it was thermally sealed over the cavity while the cavity with the folded diaper therein was positioned in the vacuum chamber and a vacuum pressure in a range of about 20 to about 25 inches of Mercury relative to the existing atmospheric pressure was removed from the chamber. The chamber was then returned to existing atmospheric pressure, which resulted in compression of the package and the compact folded absorbent article therein. The packaged article was then removed from the chamber.

The dimensions of the S-folded diaper in its package were the compared to the unfolded dimensions of the Step 3 diaper disclosed in Example 4, which was measured as described in detail in Example 1A. The dimensions of the S-folded diaper in its package were then measured. The width was 4.250 inches and the length was 2.500 inches. The area of the perimeter or "footprint" was 10.625 inches². The depth was 0.750 inches. The volume was 7.969 inches³.

The ratio of the footprint of the S folded vacuum packaged diaper compared to its unfolded footprint was 0.06. The measurement did not include any portion of the package which extended beyond the perimeter of the diaper, but only the diaper contained within. As compared to the S folded but un-packaged diaper of Example 4D, the present vacuum packaging diaper only had about 78 percent of the area of the unpackaged S folded diaper of Example 4D (an about 22 percent reduction in area), and only had about 55 percent of the volume of the unpackaged S folded diaper of Example 4D (an about 45 percent reduction in volume).

**TABLE 1- Diaper Dimensions**

| HUGGIES® ULTRATRIM® PREEMIE (Examples 1A-1E) | DIMENSIONS (inches) | | | AREA (inches²) | VOLUME (inches³) | *RATIO |
|---|---|---|---|---|---|---|
| | | | | | | |
| | W | L | D | | | |
| FLAT/UNFOLDED | | | | 99.59 | | |
| BIFOLD (1A) | 3.750 | 5.750 | 0.329 | 21.56 | 7.094 | 0.217 |
| TRIFOLD (1B) | 3.750 | 3.875 | 0.494 | 14.53 | 7.178 | 0.146 |
| QUADRIFOLD (1C) | 3.750 | 2.875 | 0.658 | 10.78 | 7.094 | 0.108 |
| S-FOLD (1D) | 3.750 | 2.000 | 0.988 | 7.50 | 7.410 | 0.075 |
| PRETZEL FOLD (1E) | 3.750 | 1.500 | 1.317 | 5.63 | 7.408 | 0.056 |
| | | | | | | |

| HUGGIES® ULTRATRlM® STEP 1 (Examples 2A-2E) | DIMENSIONS | | | AREA (in²) | VOLUME (in³) | *RATIO |
|---|---|---|---|---|---|---|
| | W | L | D | | | |
| FLAT/UNFOLDED | | | | 128.61 | | |
| BIFOLD (2A) | 4.000 | 6.625 | 0.343 | 26.50 | 9.100 | 0.206 |
| TRIFOLD (2B) | 4.000 | 4.375 | 0.514 | 17.50 | 9.000 | 0.136 |
| QUADRIFOLD (2C) | 4.000 | 3.625 | 0.686 | 14.50 | 9.950 | 0.113 |
| S-FOLD (2D) | 4.000 | 2.500 | 1.028 | 10.00 | 10.280 | 0.078 |
| PRETZEL FOLD (2E) | 4.000 | 1.750 | 1.317 | 7.00 | 9.219 | 0.054 |
| | | | | | | |

| HUGGIES® ULTRATRIM® STEP 2 (Examples 3A-3E) | DIMENSIONS | | | AREA (in²) | VOLUME (in³) | *RATIO |
|---|---|---|---|---|---|---|
| | W | L | D | | | |
| FLAT/UNFOLDED | | | | 153.36 | | |
| BIFOLD (3A) | 4.125 | 7.500 | 0.350 | 30.94 | 10.828 | 0.202 |
| TRIFOLD (3B) | 4.125 | 4.875 | 0.525 | 20.11 | 10.557 | 0.131 |
| QUADRIFOLD (3C) | 4.125 | 3.875 | 0.700 | 15.94 | 11.189 | 0.104 |
| S-FOLD (3D) | 4.125 | 2.750 | 1.050 | 11.34 | 11.910 | 0.074 |
| PRETZEL FOLD (3E) | 4.125 | 1.875 | 1.400 | 7.73 | 10.828 | 0.050 |

| HUGGIES® ULTRATRIM® STEP 3 (Examples 4A-4E) | DIMENSIONS | | | AREA (in²) | VOLUME* (in³) | *RATIO |
|---|---|---|---|---|---|---|
| | W | L | D | | | |
| FLAT/UNFOLDED | | | | 172.6 | | |
| BIFOLD (4A) | 4.500 | 8.250 | 0.354 | 37.13 | 13.142 | 0.215 |
| TRIFOLD (4B) | 4.500 | 5.375 | 0.531 | 24.19 | 12.844 | 0.140 |
| QUADRIFOLD (4C) | 4.500 | 4.000 | 0.708 | 18.00 | 12.744 | 0.104 |
| S-FOLD (4D) | 4.500 | 3.000 | 1.063 | 13.50 | 14.351 | 0.078 |
| PRETZEL FOLD (4E) | 4.500 | 1.875 | 1.417 | 8.44 | 11.956 | 0.049 |
| | | | | | | |

| HUGGIES® ULTRATRIM® STEP 4 (Examples 5A-5E) | DIMENSIONS | | | AREA (in²) | VOLUME (in³) | *RATIO |
|---|---|---|---|---|---|---|
| | W | L | D | | | |
| FLAT/UNFOLDED | | | | 208.41 | | |
| BIFOLD (5A) | 4.0625 | 9.375 | 0.375 | 38.09 | 14.282 | 0.183 |
| TRIFOLD (5B) | 4.0625 | 6.375 | 0.563 | 25.90 | 14.580 | 0.124 |
| QUADRIFOLD (5C) | 4.0625 | 4.625 | 0.750 | 18.79 | 14.091 | 0.090 |
| S-FOLD (5D) | 4.0625 | 3.375 | 1.125 | 13.71 | 14.425 | 0.066 |
| PRETZEL FOLD (5E) | 4.0625 | 2.250 | 1.500 | 9.14 | 13.711 | 0.044 |
| | | | | | | |

| HUGGIES® ULTRATRIM® STEP 5 (Examples 6A-6E) | DIMENSIONS | | | AREA (in²) | VOLUME (in³) | *RATIO |
|---|---|---|---|---|---|---|
| | W | L | D | | | |
| FLAT/UNFOLDED | | | | 231.59 | | |
| BIFOLD (6A) | 4.375 | 9.625 | 0.383 | 42.11 | 16.128 | 0.182 |
| TRIFOLD (6B) | 4.375 | 6.125 | 0.575 | 26.80 | 15.408 | 0.116 |
| QUADRIFOLD (6C) | 4.375 | 4.750 | 0.767 | 20.78 | 15.939 | 0.090 |
| S-FOLD (6D) | 4.375 | 3.250 | 1.150 | 14.22 | 16.352 | 0.061 |
| PRETZEL FOLD (6E) | 4.375 | 2.375 | 1.534 | 10.39 | 15.939 | 0.045 |
| | | | | | | |

| HUGGIES® ULTRATRIM® STEP 6 (Examples 7A-7E) | DIMENSIONS | | | AREA (in²) | VOLUME (in³) | *RATIO |
|---|---|---|---|---|---|---|
| | W | L | D | | | |
| FLAT/UNFOLDED | | | | 265.52 | | |
| BIFOLD (7A) | 4.500 | 10.375 | 0.399 | 46.69 | 18.628 | 0.176 |
| TRIFOLD (7B) | 4.500 | 6.000 | 0.599 | 27.00 | 16.173 | 0.102 |
| QUADRIFOLD (7C) | 4.500 | 5.250 | 0.798 | 23.63 | 18.853 | 0.089 |
| S-FOLD (7D) | 4.500 | 3.750 | 1.197 | 16.88 | 20.199 | 0.064 |
| PRETZEL FOLD (7E) | 4.500 | 2.500 | 1.596 | 11.25 | 17.955 | 0.042 |

| | | | | | | |
|---|---|---|---|---|---|---|
| *Folded to unfolded area | | | | | | |

### Example 1A

A diaper (HUGGIES® ULTRATRIM® diaper, available from Kimberly-Clark Corporation, Neenah, WI) was produced which is similar to the article illustrated in Figure 1, and described in detail herein. The diaper was a "preemie" size, configured to fit a premature infant. The diaper was positioned in its unfolded configuration, e.g., it's laid flat with unretracted elastics and extended to ungathered length configuration, and, as disclosed in Table 1, the diaper was measured to have an area or "footprint" of 99.59 inches². The area was determined as described below.

A rectangular sheet of translucent paper large enough to cover the absorbent article was obtained and utilized. The length and width of the sheet was measured to the nearest 0.01 inch. The paper was then weighed to the nearest 0.001 gram. The weight measurement was divided by the area measurement to determine the basis weight of the paper. The article, in this instance, the diaper, was opened completely, including any fasteners, and positioned in the laid flat configuration described above, on a planar surface. The article was taped to the surface using masking tape. For other disposable absorbent articles, such as training pants and adult incontinence pants, the articles were cut on each side between the leg openings and the waist opening prior to being positioned and taped to the planar surface. The sheet was positioned over and upon the disposable absorbent article, and a tracing of the outer perimeter, including fasteners, was made on the sheet. The paper was then cut along the traced line and weighed. The weight of the paper was multiplied by the inverse of the basis weight obtained previously. The result was an estimate of the total square inches of the absorbent article, which was reported to the nearest 0.01 in².

The diaper was then folded into the bifolded configuration illustrated in Figure 4 by hand. The dimensions of the bifolded diaper were then measured. That is, the diaper was positioned on a planar surface and restrained by hand, if necessary, in the folded position, while the length measurement 138 and the width measurement 140 were obtained and recorded.

The depth measurement 142 was obtained by measuring a standard packaged bag of articles, that is, in this instance, a bag containing bifolded diapers. The height of the bag was divided by the total number of panels (for example, in a bag of 24 bifolded diapers, there are 48 panels; diapers in the bag were packaged in the bag horizontally relative to the top height of the bag). These numbers were recorded. All measurements relating to diapers are in Tables 1 and 4.

As disclosed in Table 1, the width was 3.750 inches and the length was 5.750 inches. The area or "footprint" was 21.56 inches². The calculated depth was 0.329 inches². The volume was 7.094 inches³. The ratio of the footprint of the bifolded diaper compared to its unfolded footprint was 0.217.

### Example 1 B

A diaper (HUGGIES® ULTRATRIM®) was produced which is similar to the article illustrated in Figure 1, and described in detail herein. The diaper was a "preemie", configured to fit a premature infant. The diaper was positioned in its completely unfolded configuration, as described in Example 1A. As disclosed in Table 1, the diaper was measured to have an area or "footprint," of 99.59 inches². The diaper was folded into the trifolded configuration illustrated in Figure 5 by hand, and was measured in the same manner as described in Example 1A.

As disclosed in Table 1, the width was 3.750 inches and the length was 3.875 inches. The area or "footprint" was 14.53 inches². The calculated depth was 0.494. inches. The volume was 7.178 inches³. The ratio of the footprint of the trifolded diaper compared to its unfolded footprint was 0.146.

### Example 1C

A diaper (HUGGIES® ULTRATRIM®) was produced which is similar to the article illustrated in Figure 1, and described in detail herein. The diaper was a "preemie" size, configured for a premature infant. The diaper was positioned in its completely unfolded configuration, as described in Example 1A. As disclosed in Table 1, the diaper was measured to have an area or "footprint" of 99.59² inches. The diaper was folded into the quadrifolded configuration illustrated in Figure 6 by hand, and was measured in the same manner as described in Example 1A.

As disclosed in Table 1, the width was 3.750 inches and the length was 2.875 inches. The area or "footprint" was 10.78 inches². The calculated depth was 0.658 inches. The volume was 7.094 inches³. The ratio of the footprint of the quadrifolded diaper compared to its unfolded footprint was 0.108.

### Example 1 D

A diaper (HUGGIES® ULTRATRIM®) was produced which is similar to the article illustrated in Figure 1, and described in detail herein. The diaper was a "preemie" size, configured for a premature infant. The diaper was positioned in its completely unfolded configuration, and as disclosed in Example 1A. As disclosed in Table 1, the diaper was measured to have an area or "footprint" of 99.59 inches². The diaper was folded into the "S" folded configuration illustrated in Figure 7 by hand, and was measured in the same manner as described in Example 1A.

As disclosed in Table 1, the width was 3.75 inches and the length was 2.000 inches. The area or "footprint" was 7.50 inches². The calculated depth was 0.988 inches. The volume was 7.410 inches³. The ratio of the footprint of the "S" folded diaper compared to its unfolded footprint was 0.075.

### Example 1 E

A diaper (HUGGIES® ULTRATRIM®) was produced which is similar to the article illustrated in Figure 1, and described in detail herein. The diaper was a "preemie" size, configured for a premature infant. The diaper was positioned in its completely unfolded configuration, as described in Example 1A. As disclosed in Table 1, the diaper was measured to have an area or "footprint" of 99.59² inches. The diaper was folded into the "Pretzel" folded configuration illustrated in Figure 8 by hand, and was measured in the same manner as described in Example 1A.

As disclosed in Table 1, the width was 3.750 inches and the length was 1.500 inches. The area or "footprint" was 5.63 inches². The calculated depth was 1.317 inches. The volume was 7.408 inches³. The ratio of the footprint of the "Pretzel" folded diaper compared to its unfolded footprint was 0.056.

### Example 2A

A diaper (HUGGIES® ULTRATRIM®) was produced which is similar to the article illustrated in Figure 1, and described in detail herein. The diaper was a "Step 1" size, configured to fit an infant having a weight of about 8 lbs. to about 14 Ibs. The diaper was positioned in its completely unfolded configuration, as described in Example 1A. As disclosed in Table 1, the diaper was measured to have an area or "footprint" of 128.61 inches². The diaper was folded into the bifolded configuration illustrated in Figure 4 by hand, and it was measured in the same manner as described in Example 1A.

As disclosed in Table 1, the width was 4.000 inches and the length was 6.625 inches. The area or "footprint" was 26.50 inches². The calculated depth was 0.343 inches. The volume was 9.100 inches³. The ratio of the footprint of the bifolded diaper compared to its unfolded footprint was 0.206.

### Example 2B

A diaper (HUGGIES® ULTRATRIM®) was produced which is similar to the article illustrated in Figure 1, and described in detail herein. The diaper was a "Step 1" size, configured to fit an infant having 8 weight of about 8 lbs. to about 14 Ibs. The diaper was positioned in its completely, unfolded configuration, as described in Example 1A. As disclosed in Table 1, the diaper was measured to have an area or "footprint" of 128.61 inches² . The diaper was folded into the trifolded configuration illustrated in Figure 5 by hand, and it was measured in the same manner as described in Example 1A.

As disclosed in Table 1, the width was 4.000 inches and the length was 4.375 inches. The area or "footprint" was 17.50 inches². The calculated depth was 0.514 inches. The volume was 9.000 inches³. The ratio of the footprint of the trifolded diaper compared to its unfolded footprint was 0.136.

### Example 2C

A diaper (HUGGIES® ULTRATRIM®) was produced which is similar to the article illustrated in Figure 1, and described in detail herein. The diaper was a "Step 1" size, configured to fit an infant having a weight of about 8 lbs. to about 14 Ibs. The diaper was positioned in its completely unfolded configuration, as described in Example 1A. As disclosed in Table 1, the diaper was measured to have an area or "footprint" of 128.61 inches². The diaper was folded into the quadrifolded configuration illustrated in Figure 6 by hand, and it was measured in the same manner as described in Example 1A.

As disclosed in Table 1, the width was 4.000 inches and the length was 3.625 inches. The area or "footprint" was 14.50 inches². The calculated depth was 0.686 inches. The volume was 9.950 inches³. The ratio of the footprint of the quadrifolded diaper compared to its unfolded footprint was 0.113.

### Example 2D

A diaper (HUGGIES® ULTRATRIM®) was produced which is similar to the article illustrated in Figure 1, and described in detail herein. The diaper was a "Step 1" size, configured to fit an infant having a weight of about 8 lbs. to about 14 lbs. The diaper was positioned in its completely unfolded configuration, described in Example 1A. As disclosed in Table 1, the diaper was measured to have an area or "footprint" of 128.61 inches². The diaper was folded into the "S" folded configuration illustrated in Figure 7 by hand, and it was measured in the same manner as described in Example 1A.

As disclosed in Table 1, the width was 4.000 inches and the length was 2.500 inches. The area or "footprint" was 10.000 inches². The calculated depth was 1.028 inches. The volume was 10.280³. The ratio of the footprint of the "S" folded diaper compared to its unfolded footprint was 0.078.

### Example 2E

A diaper (HUGGIES® ULTRATRIM®) was produced which is similar to the article illustrated in Figure 1, and described in detail herein. The diaper was a "Step 1" size, configured to fit an infant having a weight of about 8 lbs. to about 14 Ibs. The diaper was positioned in its completely unfolded configuration, as described in Example 1A. As disclosed in Table 1, the diaper was measured to have an area or "footprint" of 128.61 inches² . The diaper was folded into the "Pretzel" folded configuration illustrated in Figure 8 by hand, and it was measured in the same manner as described in Example 1A.

As disclosed in Table 1, the width was 4.000 inches and the length was 1.750 inches. The area or "footprint" was 7.00 inches². The calculated depth was 1.317 inches. The volume was 9.219 inches³. The ratio of the footprint of the "Pretzel" folded diaper compared to its unfolded footprint was 0.054.

### Example 3A

A diaper (HUGGIES® ULTRATRIM®) was produced which is similar to the article illustrated in Figure 1, and described in detail herein. The diaper was a "Step 2" size, configured to fit an infant having a weight of about 12 lbs. to about 18 Ibs. The diaper was positioned in its completely unfolded configuration, as described in Example 1A. As disclosed in Table 1, the diaper was measured to have an area or "footprint" of 153.36 inches². The diaper was folded into the bifolded configuration illustrated in Figure 4 by hand, and it was measured in the same manner as described in Example 1A.

As disclosed in Table 1, the width was 4.125 inches and the length was 7.500 inches. The area or "footprint" was 30.94 inches². The calculated depth was 0.350 inches. The volume was 10.828 inches³. The ratio of the footprint of the bifolded diaper compared to its unfolded footprint was 0.202.

### Example 3B

A diaper (HUGGIES® ULTRATRIM®) was produced which is similar to the article illustrated in Figure 1, and described in detail herein. The diaper was a "Step 2" size, configured to fit an infant having a weight of about 12 lbs. to about 18 lbs. The diaper was positioned in its completely unfolded configuration, as described in Example 1A. As disclosed in Table 1, the diaper was measured to have an area or "footprint" of 153.36 inches². The diaper was folded into the trifolded configuration illustrated in Figure 5 by hand, and it was measured in the same manner as described in Example 1A.

As disclosed in Table 1, the width was 4.125 inches and the length was 4.875 inches. The area of the perimeter or "footprint" was 20.11 inches². The calculated depth was 0.525 inches. The volume was 10.557 inches³. The ratio of the footprint of the trifolded diaper compared to its unfolded footprint was 0.131.

### Example 3C

A diaper (HUGGIES® ULTRATRIM®) was produced which is similar to the article illustrated in Figure 1, and described in detail herein. The diaper was a "Step 2" size, configured to fit an infant having a weight of about 12 lbs. to about 18 lbs. The diaper was positioned in its unfolded configuration, as described in Example 1A. As disclosed in Table 1, the diaper was measured to have an area or "footprint" of 153.36 inches². The diaper was folded into the quadrifolded configuration illustrated in Figure 6 by hand, and it was measured in the same manner as described in Example 1A.

As disclosed in Table 1, the width was 4.125 inches and the length was 3.875 inches. The area or "footprint" was 15.94 inches². The calculated depth was 0.700 inches. The volume was 11.189 inches³. The ratio of the footprint of the quadrifolded diaper compared to its unfolded footprint was 0.104.

### Example 3D

A diaper (HUGGIES® ULTRATRIM®) was produced which is similar to the article illustrated in Figure 1, and described in detail herein. The diaper was a "Step 2" size, configured to fit an infant having a weight of about 12 lbs. to about 18 lbs. The diaper was positioned in its completely unfolded configuration, as described in Example 1A. As disclosed in Table 1, the diaper was measured to have an area or "footprint" of 153.36 inches². The diaper was folded into the "S" folded configuration illustrated in Figure 7 by hand, and it was measured in the same manner as described in Example 1A.

As disclosed in Table 1, the width was 4.125 inches and the length was 2.750 inches. The area or "footprint" was 11.34 inches². The calculated depth was 1.050 inches. The volume was 11.910 inches³. The ratio of the footprint of the "S" folded diaper compared to its unfolded footprint was 0.074.

### Example 3E

A diaper (HUGGIES® ULTRATRIM®) was produced which is similar to the article illustrated in Figure 1, and described in detail herein. The diaper was a "Step 2" size, configured to fit an infant having a weight of about 12 lbs. to about 18 lbs. The diaper was positioned in its completely unfolded configuration, as described in Example 1A. As disclosed in Table 1, the diaper was measured to have an area or "footprint" of 153.36 inches². The diaper was folded into the "Pretzel" folded configuration illustrated in Figure 8 by hand, and it was measured in the same manner as described in Example 1A.

As disclosed in Table 1, the width was 4.125 inches and the length was 1.875 inches. The area or "footprint" was 7.73 inches². The calculated depth was 1.400 inches. The volume was 10.828 inches³. The ratio of the footprint of the "Pretzel" folded diaper compared to its unfolded footprint was 0.050.

### Example 4A

A diaper (HUGGIES® ULTRATRIM®) was produced which is similar to the article illustrated in Figure 1, and described in detail herein. The diaper was a "Step 3" size, configured to fit an infant having a weight of about 16 lbs. to about 28 lbs. The diaper was positioned in its completely unfolded configuration, as described in Example 1A. As disclosed in Table 1, the diaper was measured to have an area or "footprint" of 172.6 inches². The diaper was folded into the bifolded configuration illustrated in Figure 4 by hand, and it was measured in the same manner as described in Example 1A.

As disclosed in Table 1, the width was 4.500 inches and the length was 8.250 inches. The area or "footprint" was 37.13 inches². The calculated depth was 0.354 inches. The volume was 13.142 inches³. The ratio of the footprint of the bifolded diaper compared to its unfolded footprint was 0.215.

### Example 4B

A diaper (HUGGIES® ULTRATRIM®) was produced which is similar to the article illustrated in Figure 1, and described in detail herein. The diaper was a "Step 3" size, configured to fit an infant having a weight of about 16 lbs. to about 28 lbs. The diaper was positioned in its completely unfolded configuration, as described in Example 1A. As disclosed in Table 1, the diaper was measured to have an area or "footprint" of 172.6 inches². The diaper was folded into the trifolded configuration illustrated in Figure 5 by hand, and it was measured in the same manner as described in Example 1A.

As disclosed in Table 1, the width was 4.500 inches and the length was 5.375 inches. The area or "footprint" was 24.19 inches². The calculated depth was 0.531 inches. The volume was 12.844 inches³. The ratio of the footprint of the trifolded diaper compared to its unfolded footprint was 0.140.

### Example 4C

A diaper (HUGGIES® ULTRATRIM®) was produced which is similar to the article illustrated in Figure 1, and described in detail herein. The diaper was a "Step 3" size, configured to fit an infant having a weight of about 16 lbs. to about 28 Ibs. The diaper was positioned in its completely unfolded configuration, as described in Example 1A. As disclosed in Table 1, the diaper was measured to have an area or "footprint" of 172.6 inches². The diaper was folded into the quadrifolded configuration illustrated in Figure 6 by hand, and it was measured in the same manner as described in Example 1A.

As disclosed in Table 1, the width was 4.500 inches and the length was 4.000 inches. The area or "footprint" was 18.0 inches². The depth was 0.708 inches. The volute was 12.744 inches³. The ratio of the footprint of the quadrifolded diaper compared to its unfolded footprint was 0.104.

### Example 4D

A diaper (HUGGIES® ULTRATRIM®) was produced which is similar to the article illustrated in Figure 1, and described in detail herein. The diaper was a "Step 3" size, configured to fit an infant having a weight of about 16 lbs. to about 28 lbs: The diaper was positioned in its completely unfolded configuration, as described in Example 1A. As disclosed in Table 1, the diaper was measured to have an area or "footprint" of 172.6 inches². The diaper was folded into the "S" folded configuration illustrated in Figure 7 by hand, and it was measured in the same manner as described in Example 1A.

As disclosed in Table 1, the width was 4.500 inches and the length was 3.000 inches. The area or "footprint" was 13.50 inches². The calculated depth was 1.063 inches. The volume was 14.351 inches³. The ratio of the footprint of the "S" folded diaper compared to its unfolded footprint was 0.078.

### Example 4E

A diaper (HUGGIES® ULTRATRIM®) was produced which is similar to the article illustrated in Figure 1, and described in detail herein. The diaper was a "Step 3" size, configured to fit an infant having a weight of about 16 lbs. to about 28 ls. The diaper was positioned in its completely unfolded configuration, as described in Example 1A. As disclosed in Table 1, the diaper was measured to have an area or "footprint" of 172.6 inches². The diaper was folded into the "Pretzel" folded configuration illustrated in Figure 8 by hand, and it was measured in the same manner as described in Example 1A.

As disclosed in Table 1, the width was 4.500 inches and the length was 1.875 inches. The area or "footprint" was 8.44 inches². The calculated depth was 1.417 inches. The volume was 11.958 inches³. The ratio of the footprint of the "Pretzel" folded diaper compared to its unfolded footprint was 0.049.

### Example 5A

A diaper (HUGGIES® ULTRATRIM®) was produced which is similar to the article illustrated in Figure 1, and described in detail herein. The diaper was a "Step 4" size, configured to fit an infant having a weight of about 22 lbs. to about 37 lbs. The diaper was positioned in its completely unfolded configuration, as described in Example 1A. As disclosed in Table 1, the diaper was measured to have an area or "footprint" of 208.41 inches². The diaper was folded into the bifolded configuration illustrated in Figure 4 by hand, and it was measured in the same manner as described in Example 1A.

As disclosed in Table 1, the width was 4.0625 inches and the length was 9.375 inches. The area or "footprint" was 38.09 inches². The calculated depth was 0.375 inches. The volume was 14.282 inches³. The ratio of the footprint of the bifolded diaper compared to its unfolded footprint was 0.183.

### Example 5B

A diaper (HUGGIES® ULTRATRIM®) was produced which is similar to the article illustrated in Figure 1, and described in detail herein. The diaper was a "Step 4" size, configured to fit an infant having a weight of about 22 lbs. to about 37 lbs. The diaper was positioned in its completely unfolded configuration, as described in Example 1A. As disclosed in Table 1, the diaper was measured to have an area or "footprint" of 208.41 inches². The diaper was folded into the trifolded configuration illustrated in Figure 5 by hand, and it was measured in the same manner as described in Example 1A.

As disclosed in Table 1, the width was 4.0625 inches and the length was 6.375 inches. The area or "footprint" was 25.90 inches². The calculated depth was 0.563 inches. The volume was 14.580 inches³. The ratio of the footprint of the trifolded diaper compared to its unfolded footprint was 0.124.

### Example 5C

A diaper (HUGGIES® ULTRATRIM®) was produced which is similar to the article illustrated in Figure 1, and described in detail herein. The diaper was a "Step 4" size, configured to fit an infant having a weight of about 22 lbs. to about 37 lbs. The diaper was positioned in its completely unfolded configuration, as described in Example 1A. As disclose in Table 1, the diaper was measured to have an area or "footprint" of 208.41 inches². The diaper was folded into the quadrifolded configuration illustrated in Figure 6 by hand, and it was measured in the same manner as described in Example 1A.

As disclosed in Table 1, the width was 4.0625 inches and the length was 4.625 inches. The area or "footprint" was 18.79 inches². The calculated depth was 0.750 inches. The volume was 14.091 inches³. The ratio of the footprint of the quadrifolded diaper compared to its unfolded footprint was 0.090.

### Example 5D

A diaper (HUGGIES® ULTRATRIM®) was produced which is similar to the article illustrated in Figure 1, and described in detail herein. The diaper was a "Step 4" size, configured to fit an infant having a weight of about 22 lbs. to about 37 lbs. The diaper was positioned in its completely unfolded configuration, as described in Example 1A. As disclosed in Table 1, the diaper was measured to have an area or "footprint" of 208.41 inches². The diaper was folded into the "S" folded configuration illustrated in Figure 7 by hand, and it was measured in the same manner as described in Example 1A.

As disclosed in Table 1, the width was 4.0625 inches and the length was 3.375 inches. The area or "footprint" was 13.71 inches². The calculated depth was 1.125 inches. The volume was 14.425 incheS³. The ratio of the footprint of the "S" folded diaper compared to its unfolded footprint was 0.066.

### Example 5E

A diaper (HUGGIES® ULTRATRIM®) was produced which is similar to the article illustrated in Figure 1, and described in detail herein. The diaper was a "Step 4" size, configured to fit an infant having a weight of about 22 lbs. to about 37 lbs. The diaper was positioned in its completely unfolded configuration, as described in Example 1A. As disclosed in Table 1, the diaper was measured to have an area or "footprint" of 208.41 inches². The diaper was folded into the "Pretzel" folded configuration illustrated in Figure 8 by hand, and it was measured in the same manner as described in Example 1A.

As disclosed in Table 1, the width was 4.0625 inches and the length was 2.250 inches. The area or "footprint" was 9.14 inches². The catenated depth was 1.500 inches. The volume was 13.711 inches³. The ratio of the footprint of the "Pretzel" folded diaper compared to its unfolded footprint was 0.044.

### Example 6A

A diaper (HUGGIES® ULTRATRIM®) was produced which is similar to the article illustrated in Figure 1, and described in detail herein. The diaper was a "Step 5" size, configured to fit an infant having a weight of over 27 Ibs. The diaper was positioned in its completely unfolded configuration, as described in Example 1 A. As disclosed in Table 1, the diaper was measured to have an area or "footprint" of 231.59 inches². The diaper was folded into the bifolded configuration illustrated in Figure 4 by hand, and it was measured in the same manner as described in Example 1A.

As disclosed in Table 1, the width was 4.375 inches and the length was 9.625 inches. The area or "footprint" was 42.11 inches². The calculated depth was 0.383inches. The volume was 16.128 inches³. The ratio of the footprint of the bifolded diaper compared to its unfolded footprint was 0.182.

### Example 6B

A diaper (HUGGIES® ULTRATRIM®) was produced which is similar to the article illustrated in Figure 1, and described in detail herein. The diaper was a "Step 5" size, configured to fit an infant having a weight of over 27 Ibs. The diaper was positioned in its completely unfolded configuration, as described in Example 1A. As disclosed in Table 1, the diaper was measured to have an area or "footprint" of 231.59 inches². The diaper was folded into the trifolded configuration illustrated in Figure 5 by hand, and it was measured in the same manner as described in Example 1A.

As disclosed in Table 1, the width was 4.375 inches and the length was 6.125 inches. The area or "footprint" was 26.80 inches². The calculated depth was 0.575. The volume was 15.408 inches³. The ratio of the footprint of the trifolded diaper compared to its unfolded footprint was 0.116.

### Example 6C

A diaper (HUGGIES® ULTRATRIM®) was produced which is similar to the article illustrated in Figure 1, and described in detail herein. The diaper was a "Step 5" size, configured to fit an infant having a weight of over 27 Ibs. The diaper was positioned in its completely unfolded configuration, as described in Example 1A. As disclosed in Table 1, the diaper was measured to have an area or "footprint" of 231.59 inches². The diaper was folded into the quadrifolded configuration illustrated in Figure 6 by hand, and it was measured in the same manner as described in Example 1A.

As disclosed in Table 1, the width was 4.375 inches and the length was 4.750 inches. The area or "footprint" was 20.78 inches². The calculated depth was 0.767 inches. The volume was 15.939 inches³. The ratio of the footprint of the quadrifolded diaper compared to its unfolded footprint was 0.090.

### Example 6D

A diaper (HUGGIES® ULTRATRIM®) was produced which is similar to the article illustrated in Figure 1, and described in detail herein. The diaper was a "Step 5" size, configured to fit an infant having a weight of over 27 Ibs. The diaper was positioned in its completely unfolded configuration, as described in Example 1A. As disclosed in Table 1, the diaper was measured to have an area or "footprint" of 231.59 inches². The diaper was folded into the "S" folded configuration illustrated in Figure 7 by hand, and it was measured in the same manner as described in Example 1A.

As disclosed in Table 1, the width was 4.375 inches and the length was 3.250 inches. The area of the perimeter or "footprint" was 14.22 inches². The calculated depth was 1.150 inches. The volume was 16.352 inches³. The ratio of the footprint of the "S" folded diaper compared to its unfolded footprint was 0.061.

### Example 6E

A diaper (HUGGIES® ULTRATRIM®) was produced which is similar to the article illustrated in Figure 1, and described in detail herein. The diaper was a "Step 5" size, configured to fit an infant having a weight of over 27 Ibs. The diaper was positioned in its completely unfolded configuration, as described in Example 1A. As discloses in Table 1, the diaper was measured to have an area or "footprint" of 231.59 inches². The diaper was folded into the "Pretzel" folded configuration illustrated in Figure 8 by hand, and it was measured in the same manner as described in Example 1A.

As disclosed in Table 1, the width was 4.375 inches and the length was 2.375 inches. The area or "footprint" was 10.39 inches². The calculated depth was 1.534 inches. The volume was 15.939 inches³. The ratio of the footprint of the "Pretzel" folded diaper compared to its unfolded footprint was 0.045.

### Example 7A

A diaper (HUGGIES® ULTRATRIM®) was produced which is similar to the article illustrated in Figure 1, and described in detail herein. The diaper was a "Step 6" size, configured to fit an infant having a weight of over 35 Ibs. The diaper was positioned in its completely unfolded configuration, as described in Example 1A. As disclosed in Table 1, the diaper was measured to have an area or "footprint" of 265.52 inches². The diaper was folded into the bifolded configuration illustrated in Figure 4 by hand, and it was measured in the same manner as described in Example 1A.

As disclosed in Table 1, the width was 4.500 inches and the length was 10.375 inches. The area or "footprint" was 46.69 inches². The calculated depth was 0.399 inches. The volume was 18.628 inches³. The ratio of the footprint of the bifolded diaper compared to its unfolded footprint was 0.176.

### Example 7B

A diaper (HUGGIES® ULTRATRIM®) was produced which is similar to the article illustrated in Figure 1, and described in detail herein. The diaper was a "Step 6" size, configured to fit an infant having a weight of over 35 Ibs. The diaper was laid flat in its completely unfolded configuration, and, as disclosed in Table 1, the diaper was measured to have an area or "footprint" of 265.52 inches². The diaper was folded into the trifolded configuration illustrated in Figure 5 by hand, and it was measured in the same manner as described in Example 1A.

As disclosed in Table 1, the width was 4.5 inches and the length was 6.00 inches. The area or "footprint" was 27.00 inches². The calculated depth was 0.599 inches. The volume was, 16.173 inches³. The ratio of the footprint of the trifolded diaper compared to its unfolded footprint was 0.102.

### Example 7C

A diaper (HUGGIES® ULTRATRIM®) was produced which is similar to the article illustrated in Figure 1, and described in detail herein. The diaper was a "Step 6" size, configured to fit an infant having a weight of over 35 Ibs. The diaper was positioned in its completely unfolded configuration, as described in Example 1A. As disclosed in Table 1, the diaper was measured to have an area or "footprint" of 265.52 inches². The diaper was folded into the quadrifolded configuration illustrated in Figure 6 by hand, and it was measured in the same manner as described in Example 1A.

As disclosed in Table 1, the width was 4.500 inches and the length was 5.250 inches. The area or "footprint" was 23.63 inches². The calculated depth was 0.798 inches. The volume was 18.853 inches³. The ratio of the footprint of the quadrifolded diaper compared to its unfolded footprint was 0.089.

### Example 7D

A diaper (HUGGIES® ULTRATRIM®) was produced which is similar to the article illustrated in Figure 1, and described in detail herein. The diaper was a "Step 6" size, configured to fit an infant having a weight of over 35 Ibs. The diaper was positioned in its completely unfolded configuration, as described in Example 1A. As disclosed in Table 1, the diaper was measured to have an area or "footprint" of 265.52 inches². The diaper was folded into the "S" folded configuration illustrated in Figure 7 by hand, and it was measured in the same manner as described in Example 1A.

As disclosed in Table 1, the width was 4.500 inches and the length was 3.750 inches. The area or "footprint" was 16.88 inches². The calculated depth was 1.197 inches. The volume was 20.199 inches³. The ratio of the footprint of the "S" folded diaper compared to its unfolded footprint was 0.064.

### Example 7E

A diaper (HUGGIES® ULTRATRIM®) was produced which is similar to the article illustrated in Figure 1, and described in detail herein. The diaper was a "Step 6" size, configured to fit an infant having a weight of over 35 Ibs. The diaper was positioned in its completely unfolded configuration, as described in Example 1A. As disclosed in Table 1, the diaper was measured to have an area or "footprint" of 265.52 inches². The diaper was folded into the "Pretzel" folded configuration illustrated in Figure 8 by hand, and it was measured in the same manner as described in Example 1A.

As disclosed in Table 1, the width was 4.500 inches and the length was 2.500 inches. The area or "footprint" was 11.25 inches². The calculated depth was 1.596 inches. The volume was 17.955 inches³. The ratio of the footprint of the "Pretzel" folded diaper compared to its unfolded footprint was 0.042.

**TABLE 2 - Training Pant Dimensions**

| HUGGIES® PULL-UPS® Training Pants (Size = Large) (Examples 8A-8D) | DIMENSIONS | | | AREA (in²) | VOLUME (in³) | *RATIO |
|---|---|---|---|---|---|---|
| | W | L | D | | | |
| FLAT/UNFOLDED | | | | 182.375 | | |
| BIFOLD (8A) | 4.375 | 10.100 | 0.532 | 44.19 | 23.508 | 0.242 |
| TRIFOLD (N/A)** | - | - | - | - | - | - |
| UADRIFOLD (8B) | 4.375 | 4.750 | 1.063 | 20.78 | 20.090 | 0.114 |
| S-FOLD (8C) | 4.375 | 3.250 | 1.595 | 14.22 | 22.679 | 0.078 |
| PRETZEL FOLD (8D) | 4.375 | 2.500 | 2.126 | 10.94 | 23.253 | 0.060 |

| | | | | | | |
|---|---|---|---|---|---|---|
| *Folded to unfolded area **The Training Pants and Adult Incontinence pants/garments were folded and measured in the same configurations as the diapers with the exception of the trifold. A seamed product can not be trifolded because of the way it is seamed. | | | | | | |

### Example 8A

A training pant (HUGGIES® PULL-UPS®, available from Kimberly-Clark Corporation, Neenah, WI) was produced which is similar to the article illustrated in Figures 1 and described in detail herein, except that it is an underwear-type of garment for a young child, as shown in Figure 12. The training pant was a "large" size. The training pant was positioned in its unfolded configuration. That is, the training pant was cut on each side from leg opening to waist opening, and laid flat with unretracted elastics and extended to ungathered length configuration, and measured as described previously in detail in Example 1A. As disclosed in Table 2, the training pant was measured to have an area or "footprint" of 182.375 inches². The area was determined as described previously in Example 1A

An identical uncut training pant, which in its configuration as underwear has a pre-existing bifold (front and back panel seamed on each side) configuration was measured. That is the training pant was positioned on a planar surface and restrained by hand, if necessary, in the position, while the length measurement 138 and the width measurement 140 were obtained and recorded.

The depth measurement 142 was obtained by measuring a standard packaged bag of articles, that is, in this instance, a bag containing training pants. The height of the bag was divided by the total number of panels in a manner substantially similar that that described previously in Example 1 A for diapers (training pants in the bag were packaged in the bag horizontally relative to the top height of the bag). These numbers were recorded. All measurements relating to training pants are in Tables 2 and 4.

As disclosed in Table 2, the width was 4.375 inches and the length was 10.100 inches. The area or "footprint" was 44.19 inches². The calculated depth was 0.532 inches². The volume was 23.508 inches³. The ratio of the footprint of the folded training pant compared to its unfolded footprint was 0.242.

### Example 8B

A training pant diaper (HUGGIES® PULL-UPS® "large" size available from Kimberly-Clark Corporation, Neenah, WI) was produced which is similar to the article illustrated in Figure 1, and described in detail herein, except that the article is an underwear-type garment for a young child., as shown in Figure 12. The training pant was positioned in its cut-open laid flat configuration, as described in Example 1A. As disclosed in Table 2, the training pant was measured to have an area or "footprint" of 182.375 inches², The training pant was folded into the quadrifolded configuration illustrated in Figure 6 by hand, and was measured in the same manner as described in Example 1A.

As disclosed in Table 2, the width was 4.375 inches and the length was 4.750 inches. The area or "footprint" was 20.78 inches². The calculated depth was 1.063 inches. The volume was 22.090 inches³. The ratio of the footprint of the folded training pant compared to its unfolded footprint was 0.114.

### Example 8C

A training pant diaper (HUGGIES® PULL-UPS® "large" size available from Kimberly-Clark Corporation, Neenah, WI) was produced which is similar to the article illustrated in Figure 1, and described in detail herein, except that the article is an underwear-type garment for a young child, as shown in Figure 12. The training pant was positioned in its cut-open laid flat configuration, as described in Example 1A. As disclosed in Table 2, the training pant was measured to have an area or "footprint" of 182.375 inches². The training pant was folded into the "S" configuration illustrated in Figure 7 by hand, and was measured in the same manner as described in Example 1A.

As disclosed in Table 2, the width was 4.375 inches and the length was 3.250 inches. The area or "footprint" was 14.22 inches². The calculated depth was 1.595 inches. The volume was 22.679 inches³. The ratio of the footprint of the folded training pant compared to its unfolded footprint was 0.078.

### Example 8D

A training pant diaper (HUGGIES® PULL-UPS® "large" size available from Kimberly-Clark Corporation, Neenah, WI) was produced which is similar to the article illustrated in Figure 1, and described in detail herein, except that the article is an underwear-type garment for a young child, as shown in Figure 12. The training pant was positioned in its cut-open laid flat configuration, as described in Example 1A. As disclosed in Table 2, the training pant was measured to have an area or "footprint" of 182.375 inches². The training pant was folded into the "Pretzel" configuration illustrated in Figure 8 by hand, and was measured in the same manner as described in Example 1A.

As disclosed in Table 2, the width was 4.375 inches and the length was 2.500 inches. The area or "footprint" was 10.94 inches². The calculated depth was 2.125 inches. The volume was 23.253 inches³. The ratio of the footprint of the folded training pant compared to its unfolded footprint was 0.060.

**TABLE 3 - Adult Incontinence Pant Dimensions**

| DEPENDS® Refastenable Under Wear (Size = Large) (Examples 9A-9D) | DIMENSIONS | | | AREA (in²) | VOLUME (in³) | *RATIO |
|---|---|---|---|---|---|---|
| | W | L | D | | | |
| FLAT/UNFOLDED | | | | 562.08 | | |
| BIFOLD (9A) | 7.125 | 15.125 | 0.499 | 107.77 | 53.775 | 0.192 |
| TRIFOLD (N/A)** | - | - | - | - | - | - |
| QUADRIFOLD (9B) | 7.125 | 10.250 | 0.998 | 73.03 | 72.885 | 0.130 |
| S-FOLD (9C) | 7.125 | 5.275 | 1.497 | 37.58 | 56.264 | 0.068 |
| PRETZEL FOLD (9D) | 7.125 | 3.625 | 1.996 | 25.83 | 51.553 | 0.046 |

| | | | | | | |
|---|---|---|---|---|---|---|
| *Folded to unfolded are **The Training Pants and Adult Incontinence pants/garments were folded and measured in the same configurations as the diapers with the exception of the trifold. A seamed product can not be trifolded because of the way it is seamed. | | | | | | |

**TABLE 4- Depth Measurement of Panel(s) of Diapers, Training Pants, and Al Pants/Garments:**

| HUGGIES® Ultratrim® Diapers Diaper Size: | Mechanical Compression^{1,2,3} |
|---|---|
| Preemie | 0.1656 |
| Step 1 | 0.1714 |
| Step 2 | 0.1750 |
| Step 3 | 0.1771 |
| Step 4 | 0.1875 |
| Step 5 | 0.1917 |
| Step 6 | 0.1995 |
| | |
| HUGGIES® | 0.2658 |
| PULL-UPS® Training Pants | |
| (Size=Large) | |
| | |
| | |
| | |
| DEPEND® | 0.2495 |
| Refastenable UnderWear | |
| | |
| (Size=Large) | |

| | |
|---|---|
| 1. This panel dimension was determined by measuring a standard bag of diapers HUGGIES® Ultratrim® diapers containing bifolded diapers and dividing the height of the bag by the total number of panels (diapers positioned horizontally relative to the top height of the bag). Using this average per panel thickness, the thickness of several fold configurations were calculated by multiplying the average per panel thickness by the number of panels in the given fold configuration. 2. This panel dimension was determined by measuring a standard bag of HUGGIES® PULL-UPS® training pants containing bifolded constructed pants and dividing the height of the bag by the total number of panels (training pants positioned horizontally relative to the top height of the bag). Using this average per panel thickness, the thickness of several fold configurations were calculated by multiplying the average per panel thickness by the number of panels in the given fold configuration. 3. This panel dimension was determine by measuring a standard bag of DEPEND® Refastenable UnderWear containing bifolded constructed garments and dividing the height of the bag by the total number of panels (garments positioned horizontally relative to the top height of the bag). Using this average per panel thickness, the thickness of several fold configurations were calculated by multiplying the average per panel thickness by the number of panels in the given fold configuration. | |

### Example 9A

An adult incontinence garment "pant" (DEPEND® Refastenable Disposable Underwear, size "large", available from Kimberly-Clark Corporation, Neenah, WI) was produced which is similar to the article illustrated in Figure 1, and described in detail herein, except that it is an underwear-type of garment for an adult, as shown in Figure 13. The garment was positioned in its unfolded configuration. That is, the underwear garment was cut on each side from leg opening to waist opening, and laid flat with unretracted elastics and extended to ungathered length configuration, and measured as described previously in detail in Example 1A. As disclosed in Table 3, the garment was measured to have an area or "footprint" of 562.08 inches². The area was determined as described previously in Example 1A.

An identical uncut garment, which in its configuration as underwear has a pre-existing bifold (front and back panel formed on each anterior and posterior side) configuration was measured. That is the garment was positioned on a planar surface and restrained by hand, if necessary, in the position, while the length measurement 138 and the width measurement 140 were obtained and recorded. The depth measurement 142 was obtained by measuring a standard packaged bag of articles, that is, in this instance, a bag containing the adult garment described herein. The height of the bag was divided by the total number of panels in a manner substantially similar that that described previously in Example 1A for diapers (the garments in the bag were packaged in the bag horizontally relative to the top height of the bag). These numbers were recorded. All measurements relating to the adult garment are in Tables 3 and 4.

As disclosed in Table 3, the width was 7.125 inches and the length was 15.125 inches. The area or "footprint" was 107.77 inches². The calculated depth was 0.499 inches². The volume was 53.775 inches³. The ratio of the footprint of the bifolded garment compared to its unfolded footprint was 0.192.

### Example 9B

An adult incontinence garment "pant" (DEFEND® Refastenable Disposable Underwear, size "large", available from Kimberly-Clark Corporation, Neenah, WI) was produced which is similar to the article illustrated in Figure 1, and described in detail herein, except that it is an underwear-type of garment for an adult, as shown in Figure 13. The garment was positioned in its cut-open laid flat configuration, as described in Example 1A. As disclosed in Table 3, the garment was measured to have an area or "footprint" of 562.08 inches². The garment was folded into the quadrifolded configuration illustrated in Figure 6 by hand, and was measured in the same manner as described in Example 1A.

As disclosed in Table 3, the width was 7.125 inches and the length was 10.250 inches. The area or "footprint" was 73.03 inches² . The calculated depth was 0.998 inches. The volume was 72.885 inches³. The ratio of the footprint of the folded garment compared to its unfolded footprint was 0.130.

### Example 9C

An adult incontinence garment "pant" (DEPEND® Refastenable Disposable Underwear, size "large", available from Kimberly-Clark Corporation, Neenah, WI) was produced which is similar to the article illustrated in Figure 1, and described in detail herein, except that it is an underwear-type of garment for an adult, as shown in Figure 13. The garment was positioned in its cut-open laid flat configuration, as described in Example 1A. As disclosed in Table 3, the garment was measured to have an area or "footprint" of 562.08 inches². The garment was folded into the "S" folded configuration illustrated in Figure 7 by hand, and was measured in the same manner as described in Example 1A.

As disclosed in Table 3, the width was 7.125 inches and the length was 5.275 inches. The area or "footprint" was 38.30 inches². The calculated depth was 1.497 inches. The volume was 56.264 inches³. The ratio of the footprint of the "S" folded garment compared to its unfolded footprint was 0.068.

### Example 9D

An adult incontinence garment "pant" (DEPEND® Refastenable Disposable Underwear, size "large", available from Kimberly-Clark Corporation, Neenah, WI) was produced which is similar to the article illustrated in Figure 1, and described in detail herein, except that it is an underwear-type of garment for an adult, as shown in Figure 13. The garment was positioned in its cut-open laid flat configuration, as described in Example 1A. As disclosed in Table 3, the garment was measured to have an area or "footprint" of 562.08 inches². The garment was folded into the "Pretzel" folded configuration illustrated in Figure 8 by hand, and was measured in the same manner as described in Example 1A.

As disclosed in Table 3, the width was 7.125 inches and the length was 3.625 inches. The area or "footprint" was 25.83 inches² . The calculated depth was 1.996 inches. The volume was 51.553 inches³. The ratio of the footprint of the folded garment compared to its unfolded footprint was 0.046.

### TEST

This invention is further illustrated by the following tests, which are not intended to limit the scope of the invention. The following test materials and test methods used to evaluate individual samples of the compressed absorbent articles of the present invention are set forth below.

A confidential, non-public test measuring absorbency of a compressed absorbent article compared to an identical non-compressed absorbent article was conducted. Nine (9) participant caregivers, each with a child using a HUGGIES® ULTRATRIM® Step 3 size (16 lbs. to 28 lbs.) diapers, were provided with fifteen (15) non-compressed HUGGIES ULTRATRIM® Step 3 diapers in a large plastic bag. The caregivers were also given fifteen (15) HUGGIES® ULTRATRIM® Step 3 diapers which had been "S" folded, as illustrated in Figure 7, and which had been vacuum packaged, similarly as described in Example 1 (the individual vacuum packaged diapers were also provided to caregivers in a large plastic bag). The caregivers tested and compared each type of diaper, i.e., the compressed individually packaged diapers against the non-compressed diapers, for three (3) days at the rate of five (5) diapers per day.

The caregivers were interviewed after the six (6) day period of testing both the currently available HUGGIES ULTRATRIM® Step 3 diapers and the compressed, vacuum packaged HUGGIES ULTRATRIM® Step 3 diapers. The participant caregivers indicated that no significant differences were noted between the un-compressed diapers and the compressed diapers.

Occasional leakage of urine and bowel movement was noted with both the un-compressed diapers and the compressed diapers. Reasonable absorbency and performance was noted with both types of diapers as well. Caregivers did not rate one type of diaper as performing better than the other; both types (un-compressed and compressed) were considered to have an equal performance relative to absorbency and leakage.

While the invention has been described in detail with respect to specific aspects thereof, it will be appreciated that those skilled in the art, upon attaining an understanding of the foregoing, may readily conceive of alterations to, variations of and equivalents to these aspects. Accordingly, the scope of the present invention should be assessed as that of the appended claims and any equivalents thereto.

## Claims

1. A vacuum packaged disposable absorbent article comprising:
a single article (10) having a folded configuration and an unfolded configuration, wherein the ratio between the area of the article in the folded configuration and the area of the article (10) in the unfolded configuration is less than 0.12 and said article (10) is a diaper (20), a training pant (200) or an adult incontinence garment (300); and
a vacuum package in which the article is positioned and sealed.

2. The vacuum packaged disposable absorbent article of claim 1, wherein the article (10) has a configuration in the package which is smaller in at least two dimensions than the un-packaged folded article (10).

3. The vacuum packaged disposable absorbent article of claims 1 or 2, wherein the article (10) is folded to have more than three overlapping folds in its folded configuration.

4. The vacuum packaged disposable absorbent article of any of claims 1 to 3 wherein the article (10) is maintained and packaged in the package at a moisture content of less than 5 percent.

5. The vacuum packaged disposable absorbent article of any preceding claim, wherein the package has a gas permeability in a range of less than about 5.0cc/100in²(645.2cm²)/24hours to about 0. 05cc/100in²(645.2cm²)/24hours.

6. The vacuum packaged disposable absorbent article of any preceding claim, configured such that a plurality of said packages may be releasably coupled together.

7. The vacuum packaged disposable absorbent article of any preceding claim, wherein the article (10) is maintained and packaged at a moisture level of less than 3 percent.

8. The vacuum packaged disposable absorbent article of any preceding claim, wherein article (10) has a configuration in the package which is at least 30 percent less in volume than an un-packaged identically folded article (10).

9. The vacuum packaged disposable absorbent article of any preceding claim, wherein the package has a gas permeability range of less than about 3.0cc/100in² (645.2cm²)/24hours to about 0.10cc/100in² (645.2cm²)/24hours.

10. The vacuum packaged disposable absorbent article of any preceding claim, wherein the package has a gas permeability range of less than about 2.0cc/100in²(645.2cm²)/24hours to about 0.10cc/100in² (645.2cm²)/24hours.

11. The vacuum packaged disposable absorbent article of any preceding claim, wherein the package is selected from the group consisting of a blister package, an injection molded package, and a form-fill-seal package.

12. The vacuum packaged disposable absorbent article of any preceding claim, wherein said ratio is less than 0.09.

## Patentansprüche

1. Ein vakuumverpackter absorbierender Einwegartikel, umfassend:
einen einzelnen Artikel (10), der eine gefaltete Konfiguration und eine entfaltete Konfiguration aufweist, wobei das Verhältnis zwischen der Fläche des Artikels in der gefalteten Konfiguration und der Fläche des Artikels (10) in der entfalteten Konfiguration weniger als 0,12 ist und der Artikel (10) eine Windel (20), ein Trainingshöschen (200) oder Erwachseneninkontinenzkleidungsstück (300) ist; und
eine Vakuumverpackung, in welcher der Artikel positioniert und versiegelt ist.

2. Der vakuumverpackte absorbierende Einwegartikel nach Anspruch 1, wobei der Artikel (10) in der Verpackung eine Konfiguration aufweist, die mindestens in zwei Dimensionen kleiner ist als der unverpackte, gefaltete Artikel (10).

3. Der vakuumverpackte absorbierende Einwegartikel nach Anspruch 1 oder 2, wobei der Artikel (10) gefaltet ist, um mehr als drei überlappende Faltungen in seiner gefalteten Konfiguration aufzuweisen.

4. Der vakuumverpackte absorbierende Einwegartikel nach einem der Ansprüche 1 bis 3, wobei der Artikel (10) in der Verpackung bei einem Feuchtigkeitsgehalt von weniger als 5 Prozent gehalten und verpackt ist.

5. Der vakuumverpackte absorbierende Einwegartikel nach einem der vorhergehenden Ansprüche, wobei die Verpackung eine Gaspermeabilität in einem Bereich von weniger als ungefähr 5,0cc/100in² 2 (645,2m²)/24 Stunden bis ungefähr 0,05cc/100in²(645,2cm²)/24 Stunden aufweist.

6. Der vakuumverpackte absorbierende Einwegartikel nach einem der vorhergehenden Ansprüche, der konfiguriert ist, so dass eine Vielzahl der Verpackungen lösbar zusammengekoppelt werden kann.

7. Der vakuumverpackte absorbierende Einwegartikel nach einem der vorhergehenden Ansprüche, wobei der Artikel (10) bei einem Feuchtigkeitsgehalt von weniger als 3 Prozent gehalten und verpackt ist.

8. Der vakuumverpackte absorbierende Einwegartikel nach einem der vorhergehenden Ansprüche, wobei der Artikel (10) in der Verpackung eine Konfiguration aufweist, die mindestens 30 Prozent weniger im Volumen ist als eine unverpackter, identisch gefalteter Artikel (10).

9. Der vakuumverpackte absorbierende Einwegartikel nach einem der vorhergehenden Ansprüche, wobei die Verpackung einen Gaspermeabilitätsbereich von weniger als ungefähr 3,0cc/100in² (645,2cm²)/24 Stunden bis ungefähr 0,10cc/100in²(645,2cm²)/24 Stunden aufweist.

10. Der vakuumverpackte absorbierende Einwegartikel nach einem der vorhergehenden Ansprüche, wobei die Verpackung einen Gaspermeabilitätsbereich von weniger als ungefähr 2,0cc/100in² (645,2cm²)/24 Stunden bis ungefähr 0,10cc/100in²(645,2cm²)/24 Stunden aufweist.

11. Der vakuumverpackte absorbierende Einwegartikel nach einem der vorhergehenden Ansprüche, wobei die Verpackung aus einer Gruppe ausgewählt ist bestehend aus einer Blisterverpackung, einer Spritzgussverpackung und einer Form-Fill-Seal- Verpackung.

12. Der vakuumverpackte absorbierende Einwegartikel nach einem der vorhergehenden Ansprüche, wobei das Verhältnis kleiner als 0,09 ist.

## Revendications

1. Article absorbant jetable conditionné sous vide, comprenant :
un article (10) unique ayant une configuration pliée et une configuration dépliée, le rapport entre la superficie de l'article dans la configuration pliée et la superficie de l'article (10) dans la configuration dépliée est inférieur à 0,12, et ledit article (10) est une couche-culotte (20), une culotte d'apprentissage de la propreté (200), ou un vêtement pour l'incontinence adulte (300) ; et
un conditionnement sous vide dans lequel l'article est placé et enfermé hermétiquement.

2. Article absorbant jetable conditionné sous vide selon la revendication 1, l'article (10) ayant, dans le conditionnement, une configuration qui est plus petite dans au moins deux dimensions que l'article (10) plié non conditionné.

3. Article absorbant jetable conditionné sous vide selon la revendication 1 ou 2, l'article (10) étant plié pour avoir plus de trois plis chevauchants dans sa configuration pliée.

4. Article absorbant jetable conditionné sous vide selon l'une quelconque des revendications 1 à 3, l'article (10) étant maintenu et conditionné dans le conditionnement à une teneur en humidité inférieure à 5 %.

5. Article absorbant jetable conditionné sous vide selon l'une quelconque des revendications précédentes, dans lequel le conditionnement a une perméabilité aux gaz comprise dans une gamme allant de moins d'environ 5,0 cm³/100 pouce² (645,2 cm²)/24 heures à environ 0,05 cm³/100 pouce² (645,2 cm²) /24 heures.

6. Article absorbant jetable conditionné sous vide selon l'une quelconque des revendications précédentes, configuré de telle sorte qu'une pluralité de conditionnements peuvent être couplés les uns aux autres de manière libérable.

7. Article absorbant jetable conditionné sous vide selon l'une quelconque des revendications précédentes, l'article (10) étant maintenu et conditionné à une teneur en humidité inférieure à 3 %.

8. Article absorbant jetable conditionné sous vide selon l'une quelconque des revendications précédentes, l'article (10) ayant, dans le conditionnement, une configuration dont le volume qui est inférieur d'au moins 30% à celui de l'article (10) plié de même façon, non conditionné.

9. Article absorbant jetable conditionné sous vide selon l'une quelconque des revendications précédentes, dans lequel le conditionnement a une gamme de perméabilité aux gaz allant de moins de 3,0 cm³/100 pouce² (645,2 cm²) /24 heures à environ 0,10 cm³/100 pouce² (645,2 cm²)/24 heures.

10. Article absorbant jetable conditionné sous vide selon l'une quelconque des revendications précédentes, dans lequel le conditionnement a une gamme de perméabilité aux gaz allant de moins de 2,0 cm³/100 pouce² (645,2 cm²)/24 heures à environ 0,10 cm³/100 pouce² (645,2 cm²)/24 heures.

11. Article absorbant jetable conditionné sous vide selon l'une quelconque des revendications précédentes, dans lequel le conditionnement est sélectionné dans le groupe consistant en un conditionnement sous coque plastique, un conditionnement moulé par injection ou un conditionnement issu de la technologie Form Fill Seal.

12. Article absorbant jetable conditionné sous vide selon l'une quelconque des revendications précédentes, dans lequel ledit rapport est inférieur à 0,09.
